# DEMANDE DE BREVET EUROPEEN

(11) **EP 2 090 376 A1**
(43) Date de publication de la demande: **19.08.2009**
(21) Numéro de dépôt: 09152008.0
(22) Date de dépôt: 04.02.2009
(51) Int. Cl.: B05B 17/06, A45D 34/00

(54) **Dispositif de pulvérisation d'une composition cosmétique**

(30) Priorité: 13.02.2008 FR 0850923
(71) Demandeur: L'Oreal, 75008 Paris (FR)
(72) Inventeur: Arnaud, Pascal, 94240, L'HAY LES ROSES (FR); Collette, Annick, 94100, ST MAUR DES FOSSES (FR); Salem, Sophie, 94800, VILLEJUIF (FR); Duru, Nicolas, 75015, PARIS (FR); Prunier, Marion, 78290, CROISSY SUR SEINE (FR); Tierce, Pascal, 59910, BONDUES (FR)
(74) Mandataire: Leonard, Armelle

(57) **Abrégé**

La présente invention a pour objet un ensemble cosmétique comprenant une composition cosmétique ou dermatologique contenue à l'intérieur d'un réservoir, ladite composition étant liquide et comprenant, dans un milieu physiologiquement acceptable, au moins une phase pulvérulente, et ladite composition présentant une viscosité allant de 5 à 500 mPa.s, et un dispositif de pulvérisation de ladite composition, comprenant une sonotrode (82) étant alimentée en composition via un canal creux d'alimentation en produit à pulvériser.

## Description

La présente invention a pour objet un dispositif de pulvérisation spécifique, sous forme de spray, comprenant une composition cosmétique comprenant au moins une phase pulvérulente et présentant une viscosité spécifique.

Les produits de maquillage traditionnels tels que, par exemple, les fonds de teint, permettent d'améliorer l'esthétique en conférant plus de relief au visage, et en intensifiant leur couleur.

Ces produits présentent des textures variées allant du fluide au solide et contiennent généralement des matières colorantes pulvérulentes. Une des difficultés rencontrées par les utilisatrices est de pouvoir étaler uniformément la composition, par exemple le fond de teint sur toute la surface du visage, de manière à répartir uniformément le produit. Les compositions de textures très épaisses ou solides sont difficiles à étaler en raison de leurs viscosités élevées. Les compositions de textures fluides ne sont pas toujours appropriées pour obtenir un maquillage uniforme, ne laissant pas de marques visibles sur la peau, notamment en raison de leur mauvais étalement sur toute la surface du visage à maquiller.

Pour répondre à ces problématiques, il a été proposé de pulvériser les compositions de maquillage à l'aide de gaz liquéfié sous pression, générateur d'aérosol. De tels gaz comprennent généralement des composés organiques volatiles (COV).

La mise en place de nouvelles législations tend à imposer une diminution de la quantité de composés organiques volatiles (COV) relâchés dans l'atmosphère par les gaz générateurs d'aérosols.

Pour parvenir à cette diminution, un gaz propulseur non COV tel que le HFA 152a peut être utilisé pour substituer partiellement ou totalement les gaz propulseurs utilisés habituellement. Néanmoins, l'utilisation de ce type de gaz n'est pas autorisée dans tous les pays.

Un autre moyen de limiter l'usage des COV est de ne pas employer de gaz de propulsion, en ayant recours à un dispositif de pulvérisation équipé d'une pompe mécanique. La société Procter & Gamble a, par exemple, proposé dans ses demandes WO 2001/12137 et WO 2001/12138, un système de pulvérisation électrostatique ne nécessitant pas de gaz propulseur. Ce type de système présente l'inconvénient de ne pas permettre de pulvériser des compositions comprenant de l'eau. Ceci est gênant lorsqu'on souhaite véhiculer des actifs hydrosolubles.

Par ailleurs, la présence de matières pulvérulentes telles que les pigments ou les charges, usuelles dans les compositions cosmétiques, présente l'inconvénient de déstabiliser les compositions, notamment les émulsions, et d'augmenter significativement la viscosité de la phase qui les comprend. En effet, dans les produits cosmétiques, il est nécessaire que la phase particulaire soir dispersée de manière homogène dans la composition, pour que le produit, notamment de maquillage, puisse être déposé uniformément sur les matières kératiniques. Plus les compositions sont visqueuses, plus la phase particulaire peut être dispersée de manière stable et homogène. L'augmentation de leur viscosité rend ces compositions difficiles à pulvériser.

En outre, certains systèmes de pulvérisation ne permettent pas la diffusion de ces particules pulvérulentes qui peuvent enrayer la tête de diffusion.

Il existe donc un besoin pour un produit cosmétique comprenant des particules pulvérulentes, et pouvant être appliqué sur la peau de manière homogène, y compris lors de leur utilisation sur une large surface corporelle.

Les inventeurs ont montré qu'il était possible d'obtenir satisfaction en ces termes en pulvérisant une composition présentant une viscosité supérieure à 5 mPa.s et comprenant des particules pulvérulentes à l'aide d'un système de pulvérisation piézoélectrique.

Un tel système permet notamment d'obtenir un maquillage homogène, naturel et ne marquant pas les imperfections de relief de la peau.

En particulier, l'invention a pour objet, selon un premier aspect, un ensemble cosmétique comprenant :
i) une composition cosmétique ou dermatologique contenue à l'intérieur d'un réservoir, ladite composition étant liquide et comprenant, dans un milieu physiologiquement acceptable, au moins une phase pulvérulente, et ladite composition présentant une viscosité allant de 5 à 500 mPa.s,
ii) un dispositif de pulvérisation de ladite composition, comprenant une sonotrode (82) couplée à un transducteur piezoélectrique et dont une surface d'éjection est alimentée en composition via un tube creux en couplage fluidique avec le réservoir et avec la sonotrode.

L'invention a également pour objet, selon un deuxième aspect, un procédé de maquillage de la peau comprenant au moins une étape de pulvérisation d'une composition cosmétique ou dermatologique telle que définie précédemment au moyen du dispositif mentionné précédemment.

L'invention a encore pour objet, selon un troisième aspect, l'utilisation d'un ensemble tel que décrit précédemment, pour obtenir un dépôt, notamment de maquillage, homogène, naturel et ne marquant pas les imperfections de relief de la peau.

### Dispositif de pulvérisation piézoélectrique

L'invention a pour objet un ensemble cosmétique comprenant une composition cosmétique ou dermatologique liquide contenue à l'intérieur d'un réservoir et un dispositif de pulvérisation de ladite composition, comprenant une sonotrode (82) couplée à un transducteur piezoélectrique et dont une surface d'éjection est alimentée en composition via un tube creux en couplage fluidique avec le réservoir et avec la sonotrode...

Par système de pulvérisation piézoélectrique, on entend, au sens de la présente demande un système qui nébulise un liquide sous l'action d'une énergie ultrasonique de fréquence et de puissance appropriées, énergie produite par un matériau piézoélectrique (transducteur) excité par un signal électrique à haute fréquence.

Selon un premier mode de réalisation, le dispositif de pulvérisation piézoélectrique peut comprendre :
- une membrane perforée, les perforations de la membrane faisant communiquer l'intérieur du récipient avec l'environnement externe,
- un actionneur pour faire vibrer la membrane,
- un moyen pour amener la composition cosmétique liquide contenue dans le récipient au contact d'une surface interne de la membrane, la composition cosmétique, sous l'effet des vibrations de la membrane s'écoulant à travers les perforations jusqu'à une surface externe de la membrane d'où elle émerge sous forme de gouttelettes.

Les perforations dans la membrane ont de préférence, une conicité inversée, c'est-à-dire une surface en coupe transversale plus grande sur la surface externe de la membrane, faisant face à l'environnement externe, que sur la surface interne, faisant face à l'intérieur du récipient.

Le dispositif de pulvérisation peut comprendre en outre un moyen de décalage de pression, tel que décrit dans la demande WO95/15822, fournissant une pression réduite au liquide en contact avec la surface interne de la membrane. La pression réduite peut varier d'une pression nulle jusqu'à la pression à laquelle l'air est aspiré à travers les perforations de la membrane en contact avec la composition.

De préférence, les perforations, sur la surface externe de la membrane, ne se touchent pas.

De préférence encore, l'actionneur est un actionneur piézo-électrique, par exemple conçu pour faire vibrer la membrane dans une gamme de fréquence allant de 20 KHz à 7 MHz. L'énergie nécessaire au fonctionnement de l'actionneur piézo-électrique peut être obtenue grâce à un générateur électrique, par exemple une pile électrique, une batterie ou une cellule photovoltaïque pouvant être éventuellement couplé à un circuit électronique.

Dans le dispositif de pulvérisation défini ci-dessus, le moyen pour amener la composition cosmétique liquide à la surface de la membrane peut comprendre un mécanisme d'alimentation par capillarité, ou alternativement, un mécanisme d'alimentation à générateur de bulles ou encore une pompe de type péristaltique, à membrane, à piston, à engrenages. De tels mécanismes sont décrits par exemple dans la demande internationale WO95/15822.

Selon un mode de réalisation particulier de l'invention, toutes les perforations ont une conicité inversée, ou à l'inverse, la membrane comporte, de plus, des perforations de conicité normale.

Par perforation de conicité normale, on entend au sens de la présente invention des perforations dont la surface en coupe transversale est plus petite sur la surface externe de la membrane, faisant face à l'environnement externe, que sur la surface interne, faisant face à l'intérieur du récipient. Lorsque des perforations de conicité normale sont présentes, celles-ci sont de préférence disposées autour et à l'extérieur des perforations de conicité inversée.

Le moyen pour amener la composition cosmétique liquide à la surface de la membrane peut être conçu pour amener ladite composition sur la surface interne de ladite membrane, ou à l'inverse, être conçu pour amener ladite composition sur la surface externe de ladite membrane. De telles variantes du dispositif de pulvérisation sont décrites, par exemple, dans la demande internationale WO95/15822.

A titre d'exemple, la membrane peut être formée d'un disque circulaire d'un diamètre de 8 mm, de nickel electroformé, d'une épaisseur de 70µm possédant une pluralité de perforations. Les perforations peuvent présenter une surface en coupe transversale en forme de disque circulaire dont le diamètre varie de 4 à 150µm sur la surface externe de la membrane, faisant face à l'environnement externe, et sur la surface interne, faisant face à l'intérieur du récipient, une surface en coupe transversale en forme de disque circulaire dont le diamètre varie de 2 à 50µm, et par exemple de 10 à 20µm.

Lors de l'utilisation du dispositif, la composition cosmétique émerge sous forme de gouttelettes dont le diamètre moyen est compris de préférence entre 20 et 100µm, et de manière encore plus préférée entre 30 et 60 µm.

Des technologies correspondant à ce dispositif ont notamment été décrites dans les demandes WO 93/10910, US 5, 487, 378, FR-A-2665572 et US 4,533,082 ; US5518179 et US6113001.

Selon un second mode de réalisation, le dispositif de pulvérisation ultrasonique peut comprendre un substrat micro-usiné, une cavité dans le substrat pour contenir la substance liquide à pulvériser, des moyens d'approvisionnement du liquide dans la cavité du substrat et des moyens de sortie du liquide qui est vaporisé sous forme de gouttelettes mono-disperses sous l'action des vibrations.

Les moyens de sortie sont constitués d'au moins un canal micro-usiné dans le subsrat qui communique avec la cavité. Par exemple le substrat est en silicium. Le dispositif générateur d'onde de pression est par exemple collé sous la cavité. L'onde de pression générée permet d'expulser une quantité de liquide contenue dans la cavité à travers les canaux micro-usinés. La quantité de liquide expulsée sous forme de fines gouttelettes est une fonction de l'onde de pression générée par exemple par une céramique de type piezo-électrique.

Grâce à un tel dispositif, il est possible de déterminer la quantité exacte de liquide pulvérisé.

Des technologies correspondant à ce dispositif ont notamment été décrites dans la demande EP1149602.

Selon un troisième mode de réalisation, le dispositif de pulvérisation ultrasonique peut utiliser un coupleur (la corne) pour transmettre mécaniquement la vibration.

En particulier, le dispositif de pulvérisation ultrasonique peut être un système d'oscillateur à ultrasons comprenant :
- un coupleur de forme conique (aussi appelé frustum),
- un oscillateur piézoélectrique et
- une plaque de résonance circulaire.

Le coupleur de forme conique (frustum) comprend de préférence deux surfaces circulaires - l'une de grande section l'autre de section plus petite - et une surface conique.

L'oscillateur piézoélectrique quant-à lui se présente sous la forme d'un disque fixé sur la surface circulaire de plus grande section du coupleur, et est pourvu de deux d'électrodes circulaires fixées sur chacune de ses surfaces. Un fil conducteur d'alimentation est attaché à chacune des plaques d'électrodes. C'est l'oscillateur piézoélectrique qui génère les vibrations.

La plaque de résonance, constituée du même matériau que le coupleur, fait partie intégrante de la surface circulaire de plus petite section du coupleur.

Une gorge est formée sur la surface conique du coupleur permettant d'adapter un membre de soutien sur ledit coupleur. Le membre de soutien permet de stabiliser l'ensemble du système d'oscillateur ultrasonique, tout en lui laissant une certaine souplesse pour ne pas altérer les vibrations. Le membre de soutien présente un orifice au travers duquel passe de l'air soufflé au moyen d'un ventilateur. Le liquide à pulvériser est délivré par un canal d'approvisionnement sur la surface de la plaque de résonance circulaire, et les particules liquides pulvérisées sont entraînées par l'air soufflé.

Des technologies correspondant à ce dispositif ont notamment été décrites dans les demandes WO91/16997, US 4, 474, 326 et FR2285930

En particulier, le dispositif de pulvérisation préféré selon l'invention, comprend un tube creu relié à un canal traversant au moins en partie la sonotrode et débouchant sur une colerette d'extrémité définissant une surface d'éjection de la composition sous forme de gouttelettes, le canal débouchant sur ladite surface d'éjection à une distance non nulle d'un bord périphérique de cette dernière, et présentant une section transversale circulaire, sur toute sa longueur.

En particulier, la sonotrode est d'axe longitudinal X, et le canal débouche sur la surface d'éjection selon la même axe X.

Dans ce mode de réalisation, le canal est rectiligne selon l'axe X.

L'invention permet d'obtenir un spray conduisant à des résultats satisfaisants.

L'invention permet notamment d'avoir une efficacité de pulvérisation relativement élevée.

La collerette peut, lors des oscillations, se déformer en changeant la forme de la surface d'éjection, qui peut passer par exemple de plane au repos à concave ou convexe vers l'avant. L'amplitude de fléchissement vers l'avant ou l'arrière peut être supérieure ou égale à 5 µm par rapport au repos, étant par exemple comprise entre 5µm et 25 µm par rapport au repos, soit une amplitude totale de 10 à 50 µm.

L'épaisseur minimale de la collerette d'extrémité dans la région d'éjection des particules du produit est comprise par exemple entre 0,4 et 0,6 mm, mieux 0,45 et 0,55 µm, étant de préférence de 0,5 mm.

L'éjection des gouttelettes de produit peut avoir lieu sur toute la circonférence de la collerette d'extrémité, ce qui contribue à l'obtention d'un spray homogène.

L'invention a encore pour objet, selon un autre de ses aspects, un dispositif de pulvérisation d'un produit cosmétique ou dermatologique, comportant une sonotrode et un transducteur couplé à la sonotrode, la sonotrode présentant une collerette d'extrémité définissant une surface d'éjection des particules de produit, la sonotrode comportant également une portion de diamètre décroissant prolongée par une portion cylindrique (encore appelée trompe) se raccordant à la collerette d'extrémité,
le ratio *diamètre transducteur*/ *diamètre de la portion cylindrique* étant inférieur ou égal à 4,5, mieux à 4, encore mieux 3,7 et de préférence supérieur ou égal à 3, de préférence encore compris entre 3,5 et 3,7, et/ou
le ratio *diamètre de la collerette* / *diamètre de la portion cylindrique* étant compris entre 7/6 et 13/4, et/ou
le ratio *diamètre de la collerette* / *épaisseur de la collerette* étant compris entre 70/6, par exemple 12, et 130/4, par exemple 32.

Ces caractéristiques géométriques conduisent à des résultats particulièrement satisfaisants.

La collerette peut avoir une plus grande dimension transversale inférieure ou égale à λ/4, où λ est la longueur d'onde dans le matériau de la sonotrode de l'onde ultrasonore.

La longueur de la sonotrode, entre la face de la sonotrode au contact d'un transducteur servant à mettre en vibrations la sonotrode et la surface d'éjection peut être inférieure ou égale à λ.

Le canal d'amenée du produit peut présenter une portion rétrécie.

La section rétrécie peut freiner l'écoulement du produit et améliorer les performances de la pulvérisation. La portion rétrécie peut notamment permettre d'obtenir un spray relativement homogène.

La présence de la portion rétrécie facilite la fabrication du reste du canal, qui peut avoir une section relativement grande, ce qui limite les pertes de charge.

La portion rétrécie peut assurer une certaine retenue capillaire en l'absence d'utilisation du dispositif et permet de réduire les échanges avec l'air. L'utilisation d'un obturateur pour le canal d'amenée peut être évitée.

La sonotrode est couplée à un transducteur permettant de transformer une énergie électrique en vibrations ultrasonores. La fréquence de résonance de la sonotrode est de préférence la plus proche possible de celle du transducteur. Le couplage peut s'effectuer par exemple par collage ou vissage.

Les particules de produit sont avantageusement entraînées vers la région à traiter par un flux d'air produit par au moins un ventilateur. Le débit d'air est par exemple compris entre 4 et 7 m³/h, mieux entre 5,5 et 6,5 m³/h.

Dans un exemple de réalisation, la portion rétrécie débouche sur la surface d'éjection. La portion rétrécie peut présenter une section transversale constante sur une distance d'au moins 1 mm et inférieure ou égale à 10 mm. La longueur de la portion rétrécie est par exemple inférieure ou égale à 7 mm, mieux comprise entre 1mm et 5mm, étant par exemple de 2,5mm. La portion rétrécie peut présenter une section transversale constante depuis l'extrémité où elle débouche sur la surface d'éjection jusqu'à l'extrémité opposée.

La portion rétrécie présente avantageusement une section transversale circulaire, ce qui facilite sa réalisation.

Le canal peut présenter une section transversale circulaire, sur toute sa longueur.

Le canal est avantageusement rectiligne, de même axe longitudinal que la sonotrode. La portion rétrécie peut présenter une plus petite section transversale inférieure ou égale à 0,8 mm². La portion rétrécie peut notamment présenter un diamètre inférieur ou égal à 1 mm, par exemple compris entre 0,4 mm et 0,8 mm, de préférence voisin de 0,6 mm.

Le canal peut présenter une plus grande section transversale supérieure ou égale à 0,8 mm².

Le canal peut présenter, hors de la portion rétrécie, un diamètre compris entre 1 mm et 2 mm, par exemple voisin de 1,5 mm, voire plus important, notamment lorsque le transducteur est fixé par boulonnage sur la sonotrode.

Le ratio longueur portion rétrécie / longueur totale du canal de la sonotrode peut être compris entre 0,04 et 0,4.

Le ratio en surface section transversale la plus large du canal / section la plus étroite du canal peut être compris entre 1 et 25, notamment entre 4 et 10, par exemple 6 et 6,5.

Le canal peut alimenter la surface d'éjection par un orifice de sortie unique, qui peut être situé au centre de la surface d'éjection. Ce mode de réalisation constitue un mode préféré.

La sonotrode peut être réalisée de manière monolithique avec un embout de raccordement à un tube d'alimentation en produit du canal. Ce tube d'alimentation peut être un conduit flexible, ce qui permet l'utilisation du conduit au sein d'une pompe péristaltique. Le raccordement du canal au conduit d'alimentation peut encore se faire autrement, par exemple au moyen d'un embout inséré dans la sonotrode.

Le transducteur peut être traversé par l'embout, ayant par exemple une forme annulaire.

Le diamètre extérieur de la collerette d'extrémité est compris par exemple entre 7 et 13 mm, mieux 8 et 12 mm, encore mieux 9 et 11 mm, de préférence voisin de 10 mm. De bons résultats peuvent être obtenus, dans un exemple de réalisation, avec un diamètre de 10 mm pour la collerette d'extrémité et une épaisseur minimale de 0,5 mm pour la collerette, pour une fréquence de 100 kHz ± 10%.

La plage annulaire périphérique de la collerette où l'épaisseur de la collerette est relativement faible, notamment inférieure ou égale à 0,6 mm, peut avoir une largeur, mesurée radialement supérieure ou égale à 0,2 mm, par exemple de 0,2 mm à 2 mm.

La collerette d'extrémité peut comporter une plage annulaire faisant 0,5 mm d'épaisseur qui s'étend sur une largeur, mesurée radialement, d'au moins 0.5 mm.

La sonotrode peut présenter une portion dont la section transversale extérieure décroît en direction de la surface d'éjection, notamment une portion tronconique. L'angle au sommet de cette portion tronconique peut être compris entre 10° et 45°, notamment être de 30°.

La sonotrode peut présenter une portion cylindrique de révolution, comme mentionné plus haut. La portion de section extérieure décroissante peut se raccorder à cette portion cylindrique de révolution, la portion cylindrique de révolution étant intermédiaire entre la portion de section décroissante, notamment tronconique, et la collerette d'extrémité.

Le diamètre extérieur de la portion cylindrique de révolution est compris par exemple entre 4 et 7 mm, notamment peut être voisin de 5,5 mm.

La longueur de la portion cylindrique de révolution est comprise par exemple entre 3 et 5 mm.

La longueur des différentes portions de la sonotrode est de préférence choisie en fonction de la fréquence nominale à laquelle la sonotrode est prévue pour résonner, la surface d'éjection devant préférablement se situer sensiblement au niveau d'un ventre de vibrations. La distance séparant la face d'éjection et le transducteur ainsi que le diamètre de la collerette d'extrémité peut dépendre de la longueur d'onde λ = c/f, où c est la célérité du son dans le matériau à la température d'utilisation, et f la fréquence.

La sonotrode peut être usinée, étant de préférence réalisée en métal, notamment en aluminium ou alliage d'aluminium, en titane ou ses aliages, en inox, par exemple inox 316

La fréquence d'excitation du transducteur est comprise par exemple entre 30 et 200 kHz. Par exemple, la fréquence d'excitation peut être de l'ordre de 100 kHz ± 10 %. De préférence, la fréquence d'excitation du transducteur est comprise entre 100 et 200 kHz.

La taille moyenne des particules du spray dépend de la fréquence et des caractéristiques rhéologiques du fluide à nébuliser. La taille moyenne est, dans un exemple de mise en oeuvre de l'invention, comprise entre 20 et 25 µm, notamment à une fréquence de 100 kHz.

Le taux de particules fines de taille inférieure à 10 µm peut être inférieur à 10 %.

L'invention a encore pour objet, selon un autre de ses aspects, un dispositif de conditionnement et de pulvérisation d'un produit cosmétique ou dermatologique, comportant une tête telle que définie ci-dessus.

Ce dispositif peut comporter un récipient contenant le produit à pulvériser. Ce produit peut être un produit de soin ou de maquillage, notamment un fond de teint ou un produit comportant un agent coiffant, un auto-bronzant ou une composition de protection solaire.

Le récipient peut se présenter sous la forme d'une cartouche amovible.

Le produit peut être contenu dans une poche souple.

Le dispositif peut comporter un boîtier avec, notamment en partie supérieure, un logement pour recevoir la cartouche précitée.

Le flux d'air dirigé vers les matières kératiniques peut être chauffé ou refroidi, selon les besoins.

Dans un exemple de mise en oeuvre de l'invention, la pulvérisation est déclenchée par une action de l'utilisateur sur un organe de commande tel qu'un bouton-poussoir par exemple.

Une fois qu'un cycle de pulvérisation est déclenché, une séquence de pulvérisation comportant les étapes suivantes peut avoir lieu :
i) mise en route d'un ventilateur créant un flux d'air d'entraînement des particules de produit,
ii) après un retard prédéfini, mise en vibrations de la sonotrode par un transducteur,
iii) après un nouveau retard, mise en route d'une pompe alimentant la sonotrode en produit.

A la fin du cycle de pulvérisation, l'arrêt du dispositif peut comporter successivement l'arrêt de la pompe, l'arrêt du transducteur, et l'arrêt du ventilateur.

L'invention a encore pour objet, selon un autre de ses aspects, un dispositif de pulvérisation comportant une buse, un support disposé à l'intérieur de la buse, une sonotrode couplée à un transducteur, fixée par encliquetage sur le support, avec interposition d'un joint entre un épaulement du support et un épaulement de la sonotrode.

Cet aspect de l'invention facilite le montage de la sonotrode dans le dispositif.

L'invention a encore pour objet, selon un autre de ses aspects, un dispositif de pulvérisation comportant une sonotrode, un transducteur de forme annulaire couplé à la sonotrode, cette dernière étant réalisée de façon monolithique avec un embout sur lequel est inséré un tube d'alimentation en produit à pulvériser. Cet aspect de l'invention facilite la construction du dispositif.

L'invention a encore pour objet, selon un autre de ses aspects, un procédé de traitement cosmétique, par exemple de la peau, notamment de maquillage, ou de traitement de la chevelure, comportant l'étape consistant à pulvériser un produit cosmétique sur les matières kératiniques humaines concernées, en utilisant une tête de pulvérisation telle que définie ci-dessus.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 représente de manière schématique, en élévation, un exemple de dispositif de pulvérisation réalisé conformément à l'invention,
- la figure 2 représente le dispositif de la figure 1 avec la cartouche de produit en place, prêt à pulvériser,
- la figure 3 représente le dispositif de la figure 1 avec la trappe d'accès au logement de réception de la cartouche ouverte, en attente de la mise en place de la cartouche sur le boîtier,
- la figure 4 est une vue schématique et partielle, en perspective éclatée, du dispositif des figures 1 à 3,
- la figure 5 représente en perspective, de manière schématique et partielle, l'ensemble de pulvérisation,
- la figure 6 est une coupe longitudinale, schématique et partielle, de l'ensemble de pulvérisation de la figure 5,
- la figure 7 représente isolément le support du transducteur,
- la figure 8 représente isolément, en perspective, la sonotrode,
- la figure 9 est une vue en élévation de la sonotrode,
- la figure 10 est une coupe longitudinale de la sonotrode, selon X-X de la figure 9,
- la figure 11 représente en perspective une variante de réalisation de la sonotrode,
- la figure 12 est une coupe longitudinale schématique et partielle d'une tête de pulvérisation comportant la sonotrode de la figure 11, et
- la figure 13 représente de manière schématique, en perspective, la résistance chauffante du dispositif de chauffage.

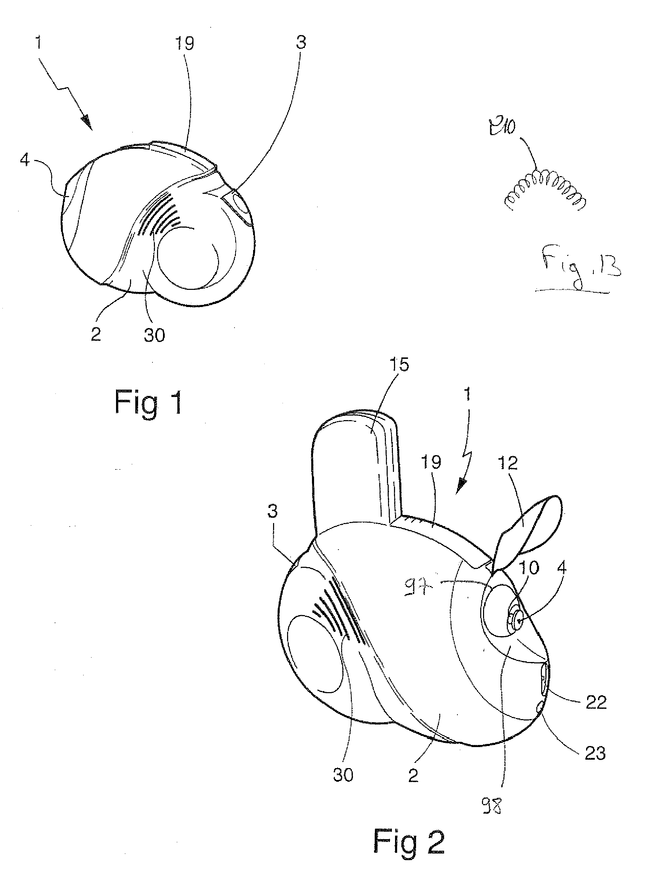

Le dispositif de pulvérisation 1 représenté sur les figures 1 à 3 comporte un boîtier 2 manipulable par l'utilisateur pour pulvériser un produit sur la peau ou d'autres matières kératiniques humaines, telles que les lèvres ou les cheveux.

Le boîtier 2 porte à l'arrière, dans l'exemple considéré, un bouton-poussoir 3 qui permet à l'utilisateur, en appuyant dessus, de déclencher la pulvérisation. Ce bouton-poussoir 3 pourrait en variante être situé ailleurs et remplacé par une gâchette ou une touche sensitive, par exemple.

Le dispositif 1 comporte à l'avant, comme on peut le voir sur la figure 2 notamment, une surface 4 d'éjection des particules de produit. Cette surface peut être orientée vers la région à traiter, lors de l'utilisation, de manière à permettre aux particules de produit de se déposer sur cette région.

Le boîtier 2 comporte dans l'exemple considéré un capot de protection 12 pouvant être rabattu devant la surface d'éjection 4 en absence d'utilisation. Ce capot 12 est par exemple articulé sur le corps du boîtier, entre une position abaissée où il recouvre la surface d'éjection 4 et une position relevée. Dans une variante de réalisation, le boîtier est dépourvu de capot de protection ou ce dernier est monté autrement dans le boîtier.

Le capot 12 peut s'étendre dans la continuité de la surface extérieure du boîtier 2, en position rabattue.

Le boîtier 2 peut accueillir une cartouche 15 contenant le produit à pulvériser, cette cartouche 15 étant introduite dans un logement 17 du boîtier 2.

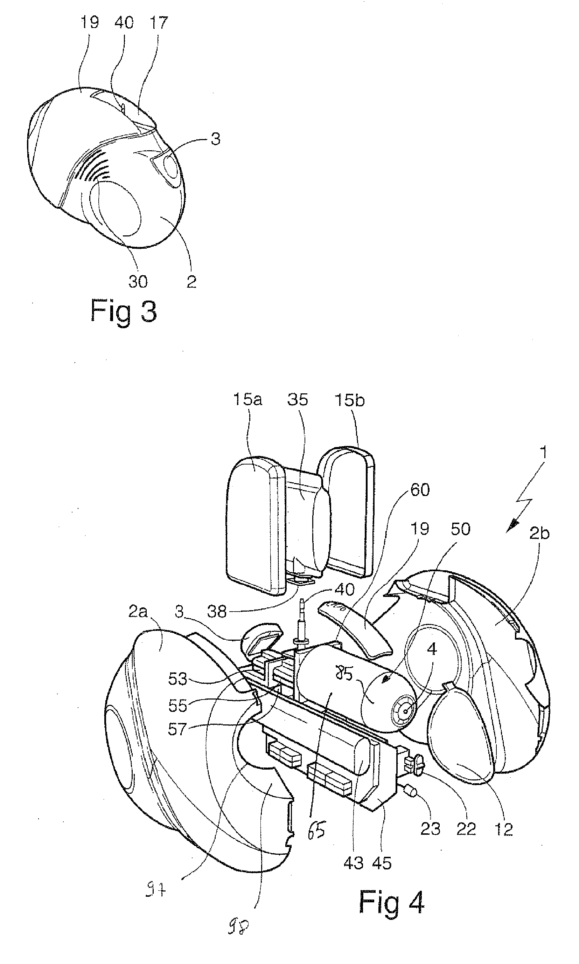

Comme on peut le voir à la figure 3, ce logement 17 peut être obturé en l'absence d'utilisation par un volet obturateur 18.

Dans l'exemple illustré, le logement 17 est ouvert vers le haut.

Le volet obturateur 18 peut être monté coulissant sur le boîtier 2. Dans des variantes non illustrées, le logement 17 est disposé autrement sur le boîtier.

Le produit contenu dans la cartouche 15 est par exemple un fond de teint, un auto-bronzant, une lotion pour le corps ou le visage ou un produit contenant un agent coiffant.

La contenance de la cartouche est par exemple comprise entre 1ml et 100ml, mieux entre 5ml et 20ml, notamment 10ml.

Dans une variante non illustrée, le dispositif 1 peut recevoir plusieurs cartouches contenant des produits différents ou une cartouche contenant plusieurs produits, avec un moyen de sélection du produit à pulvériser, ou en variante un moyen de réglage de la proportion d'un produit par rapport à l'autre dans le mélange pulvérisé. Le cas échéant, une même cartouche peut contenir plusieurs produits avec un moyen de sélection du produit devant être pulvérisé ou de réglage de la proportion des différents produits dans le mélange pulvérisé.

Le boîtier 2 comporte à l'avant, dans l'exemple considéré, un interrupteur général de marche/arrêt 22 et un voyant 23 servant de témoin de fonctionnement. Le boîtier 2 comporte, sur les côtés, des ouïes d'entrée d'air 30.

On peut voir sur la figure 4 que le corps du boîtier 2 peut être formé par l'assemblage de deux-demi coques 2a et 2b. Ces dernières sont par exemple montées avec un ajustement serré, éventuellement réversible, étant par exemple encliquetées l'une sur l'autre et/ou maintenues par des vis. Ces demi-coques 2a et 2b sont par exemple réalisées par moulage d'une matière thermoplastique.

La cartouche 15 peut comporter deux demi-coques 15a et 15b qui sont réunies autour d'une poche souple 35 contenant le produit à pulvériser, cette poche 35 étant par exemple thermosoudée sur un embout de raccordement 38 destiné à être engagé sur un embout d'aspiration 40 présent dans le logement 17.

L'utilisation d'une poche souple 35 permet un prélèvement de produit sans reprise d'air dans la poche. En variante, la cartouche peut contenir un réservoir autre qu'une poche souple, par exemple un réservoir à fond mobile.

La cartouche peut comporter, dans une variante de mise en oeuvre, un indicateur visuel du degré de vidage, par exemple une fenêtre transparente réalisée dans l'une des demi-coques 15a et 15b et/ou dans la poche souple 35.

Les demi-coques 15a et 15b sont par exemple montées avec un ajustement serré, éventuellement réversible, étant par exemple encliquetées et/ou collées l'une sur l'autre ou fixées autrement, étant par exemple réalisées dans une matière thermoplastique, opaque ou transparente.

La disposition de la cartouche amovible 15 en partie supérieure du dispositif permet de bénéficier d'un effet de gravité pour l'amenée du produit.

Le cas échéant, une cartouche d'un produit nettoyant peut être utilisée en remplacement d'une cartouche usuelle, pour nettoyer le dispositif, en particulier la sonotrode et la surface d'éjection.

Le dispositif peut être proposé à l'utilisateur, par exemple au sein d'un emballage commun, avec une ou plusieurs cartouches contenant un ou plusieurs produits à pulvériser et la cartouche de nettoyage ci-dessus.

La cartouche de nettoyage peut être rechargeable ou non.

La solution de nettoyage pourra être choisie parmi l'un des solvants de la composition cosmétique pour lui être compatible, et comporter par exemple de l'isododécane, une silicone volatile ou encore de l'alcool ou de l'eau.

Le dispositif peut comporter, le cas échéant, un système de reconnaissance de la cartouche, par exemple grâce à un palpeur électromécanique, à des contacts électriques ou à une puce RFID.

La connaissance par le dispositif 1 du contenu de la cartouche en place peut permettre d'adapter automatiquement des paramètres de fonctionnement au dispositif du produit à pulvériser, par exemple le débit de produit, la fréquence d'excitation, le débit d'air et/ou la température de l'air le cas échéant.

Le boîtier 2 loge une source d'énergie électrique 43, par exemple un ou plusieurs accumulateurs ou piles, et un circuit imprimé 45 portant les composants électroniques du dispositif 1. Ces composants assurent la génération de la tension nécessaire à la pulvérisation, le pilotage des différents éléments électriques et peuvent exécuter des fonctions annexes telles que par exemple le calcul de la quantité de produit restant à pulvériser, afin de signaler à l'utilisateur la nécessité de procéder au remplacement de la cartouche.

L'ouverture du boîtier 2 par séparation des demi-coques 2a et 2b peut être nécessaire pour remplacer les piles. En variante, l'accès au compartiment des piles peut se faire sans ouverture du boîtier, grâce à une trappe d'accès à ce compartiment. Le dispositif de pulvérisation 1 peut comporter, le cas échéant, un connecteur électrique permettant de recharger un accumulateur présent dans le boîtier.

Le boîtier 2 loge également un ensemble (encore appelé tête) de pulvérisation 50 ainsi qu'une pompe 53, cette dernière étant reliée d'une part à l'embout d'aspiration 40 et d'autre part à l'ensemble de pulvérisation 50 par un tube 55, qui est de préférence un conduit flexible.

La pompe 53 est par exemple du type péristaltique, comportant un moteur électrique 57 entraînant en rotation un ou plusieurs galets venant en appui sur le conduit flexible 55 pour pousser le produit vers l'ensemble de pulvérisation 50. Le débit de produit lors du fonctionnement de la pompe 53 va par exemple de 0,05 g/mn à 2g/mn.

Le cas échéant, le débit peut être réglable par l'utilisateur avec certaines valeurs préréglées.

Dans des variantes non illustrées, d'autres types de pompes sont utilisés, par exemple à engrenages, à membrane ou à piston. Une alimentation par gravité ou poche élastique rétractable peut encore être envisagée.

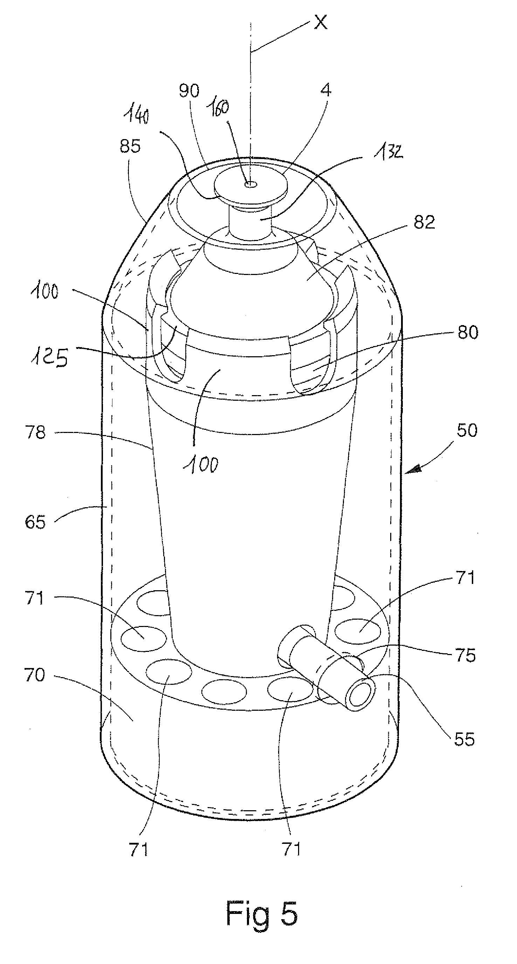

L'ensemble de pulvérisation 50 comporte, à l'arrière, un ventilateur 60, comme on peut le voir sur la figure 4, ce ventilateur 60 n'ayant pas été représenté sur la figure 5 dans un souci de clarté du dessin.

L'ensemble de pulvérisation 50 comporte également une buse 65 comportant un corps tubulaire, fermé à l'arrière par un bouchon 70 pourvu d'ouvertures 71 pour le passage de l'air soufflé par le ventilateur 60.

Le ventilateur 60 est par exemple fixé sur le bouchon 70, par exemple par des vis.

L'axe de rotation du ventilateur est par exemple confondu avec l'axe longitudinal de la buse 65.

Le débit d'air injecté dans la buse 65 par le ventilateur 60 est par exemple compris entre 4 et 7 m³/h_{.}

L'air est aspiré par le ventilateur 60 à l'extérieur du boîtier 2 grâce aux ouïes 30.

Le ventilateur 60 peut fonctionner en permanence dès que l'utilisateur a allumé le dispositif grâce à l'interrupteur général 22 ou, en variante, seulement lorsque l'utilisateur déclenche la pulvérisation, en appuyant sur le bouton-poussoir 3. Dans un exemple, le fonctionnement du ventilateur peut se prolonger après la fin de la pulvérisation pendant une durée prédéfinie ou jusqu'à une nouvelle action de l'utilisateur sur le dispositif, afin de permettre à l'utilisateur de profiter de l'air soufflé pour accélérer le séchage du produit déposé sur la région à traiter.

Toujours dans un exemple de mise en oeuvre de l'invention, un cycle de pulvérisation commandé par une action sur le bouton-poussoir 3 comprend tout d'abord la mise en route du ventilateur, puis après un retard compris entre 300 et 800 ms par exemple, par exemple de 500ms environ, la tête de pulvérisation est excitée, puis après un nouveau retard, par exemple compris entre 300 et 800 ms, notamment de l'ordre de 500 ms, la pompe 53 est mise en marche. L'arrêt de la pulvérisation s'effectue lorsque le bouton-poussoir 3 est relâché, les étapes ci-dessus se succédant dans l'ordre inverse.

Le dispositif 1 comporte avantageusement un moyen de chauffage 200 de l'air soufflé vers la surface sur laquelle le spray est pulvérisé. Cela accélère le séchage du produit et le dispositif est ainsi plus confortable à utiliser. Cela peut également réchauffer la sonotrode et réduire la viscosité du produit, facilitant son écoulement et la pulvérisation.

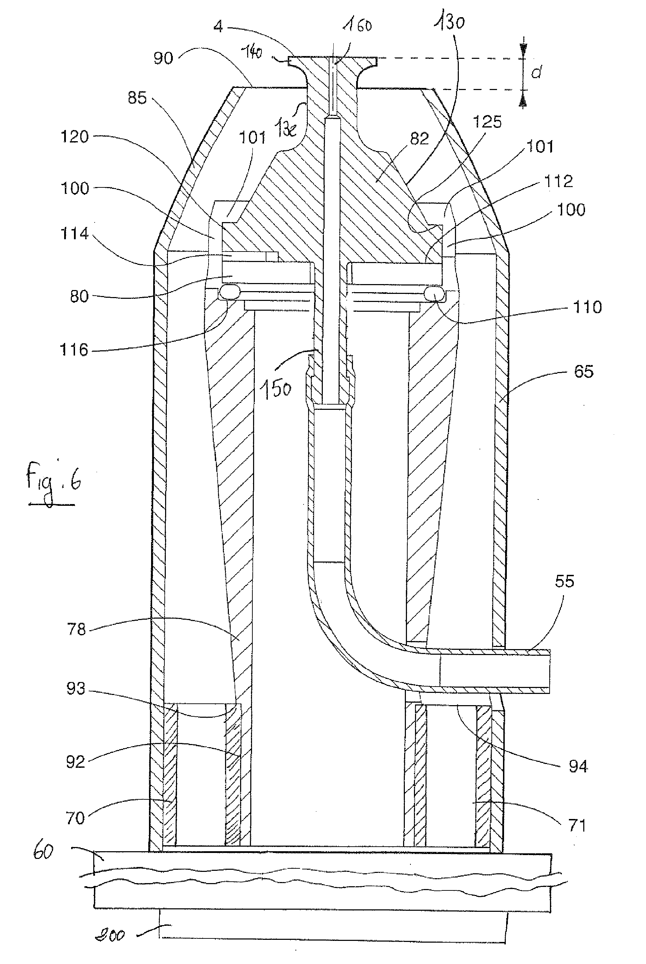

Le moyen de chauffage 200 comporte par exemple une résistance chauffante électrique 210 qui peut être intégrée au ventilateur 60 ou placée en amont ou en aval du ventilateur, de préférence en amont, comme illustré à la figure 6.

Le moyen de chauffage 200 est par exemple fixé au ventilateur 60.

Dans un exemple, la résistance chauffante 210 est constituée d'un fil de Nichrome de 0,51 mm de diamètre et de 2,8 m de longueur, enroulé sous forme de ressort comme illustré à la figure 13, placé derrière le ventilateur 60, et alimenté avec une puissance de 36 W. Une telle résistance chauffante permet de produire un flux d'air à la température de 36°C à 10 cm de la surface d'éjection du produit.

La buse 65, le ventilateur 60 et le moyen de chauffage 200 peuvent constituer un ensemble monobloc facile à monter dans le boîtier 2. De plus, l'alignement de ces éléments rend le dispositif relativement compact.

La température à laquelle l'air chaud sort de la buse 65 est par exemple comprise entre 30 et 40 °C, idéalement de 37°C environ.

La température de sortie de l'air peut être régulée, le cas échéant, grâce à la présence d'un capteur de température exposé au flux d'air chaud et d'une boucle de régulation électronique.

Le dispositif peut être agencé de manière à permettre à l'utilisateur de choisir entre un fonctionnement où l'air soufflé par le dispositif est chauffé et un fonctionnement où il ne l'est pas.

Ce choix peut s'effectuer par exemple grâce à un sélecteur actionnable par l'utilisateur, ce sélecteur étant par exemple commandé par une pression plus ou moins grande sur le bouton-poussoir déclenchant la pulvérisation.

Par exemple, une pression modérée sur le bouton-poussoir 3 déclenche la pulvérisation avec soufflage d'air à température ambiante et une pression plus importante déclenche la pulvérisation avec soufflage d'air chaud.

Le dispositif de chauffage peut s'allumer en même temps que le ventilateur est mis en marche et s'éteindre en même temps également, ou les mises en marches respectives peuvent être différées dans le temps.

Le dispositif de pulvérisation 1 peut être agencé pour passer dans un mode de veille en l'absence d'actionnement du bouton-poussoir 3 pendant une durée prédéfinie. Le retour au fonctionnement normal du dispositif peut alors nécessiter une pression sur le bouton-poussoir 3 ou l'actionnement de l'interrupteur général 22.

Le corps de la buse 65 est pourvu d'une ouverture latérale 75 pour le passage du tube 55 d'alimentation en produit, et loge un support 78 qui maintient un transducteur piézoélectrique 80.

Ce dernier est mécaniquement couplé à une sonotrode 82 permettant d'amplifier les vibrations électromécaniques du transducteur 80, lesquelles sont radiales ou longitudinales, pour les transmettre à la surface d'éjection 4, cette dernière étant définie par une collerette d'extrémité de la sonotrode 82.

Dans l'exemple considéré, celle-ci est usinée en aluminium mais d'autres matériaux, notamment d'autres métaux ou alliages, peuvent être utilisés.

La face arrière de la sonotrode 82 est collée au transducteur 80 mais la fixation pourrait encore s'effectuer autrement, notamment par des moyens mécaniques tels que vissage.

Le corps de la buse 65 est par exemple cylindrique de révolution et peut être moulé dans une matière thermoplastique.

La buse 65 peut présente, à l'avant, une portion convergente 85, se terminant par une ouverture 90 de même axe X que celui de la sonotrode 82. Cette ouverture 90 est circulaire dans l'exemple considéré, de diamètre compris entre 14 et 20 mm, par exemple de l'ordre de 16 mm.

La portion convergente 85 fait saillie dans un renfoncement 91 du boîtier 2, formé par l'assemblage des demi-coques 2a et 2b, le fond de ce renfoncement 98 définissant une ouverture 97 pouvant épouser localement la section extérieure de la buse 65.

Dans l'exemple illustré le flux d'air soufflé par la buse 65 n'est pas dévié par le reste du boîtier, le renfoncement 91 étant suffisamment large.

L'air soufflé par le ventilateur 60 sort par l'ouverture 90 selon un flux d'air dirigé généralement selon l'axe X.

Comme on peut le voir sur la figure 6 notamment, la surface d'éjection 4 fait saillie par rapport au plan P de l'ouverture 90 d'une distance d. Le plan P de l'ouverture 90 est perpendiculaire à l'axe X.

La distance d est par exemple comprise entre 2 et 4 mm, mieux 2 à 3 mm, encore mieux 2,2 et 2,9 mm, notamment pour un diamètre de l'ouverture 90 de 16 mm environ. De telles valeurs permettent d'obtenir un spray relativement homogène avec peu de pertes à 5, voire 10 cm, de distance de la surface d'éjection 4.

Une distance d hors de la plage ci-dessus peut conduire à une moins bonne homogénéité du spray, avec par exemple un manque central et/ou une tache de produit moins précise.

Le support 78, qui est par exemple moulé d'une seule pièce en une matière thermoplastique, comporte une portion 92 prévue pour s'emmancher à force dans la lumière centrale 72 du bouchon 70, jusqu'à butée d'un épaulement 93 du support 78 contre la face intérieure 94 du bouchon 70.

Le support 78 comporte, à l'opposé de la portion de montage 92, des pattes 100 élastiquement déformables, par exemple au nombre de quatre, pourvues chacune d'une dent 101 à leur extrémité, permettant le maintien par encliquetage de la sonotrode 82 et du transducteur 80, comme illustré aux figures 5 et 6.

Le support 78, outre le maintien de la sonotrode, peut contribuer également à une bonne distribution du flux d'air à l'intérieur de la buse 65, tout autour de la sonotrode 82.

Le transducteur 80, qui présente une forme annulaire, est dans l'exemple considéré pris en sandwich entre d'une part un joint torique 101 et d'autre part la face arrière 112 de la sonotrode.

Un évidement 114 est réalisé dans la face arrière 112 pour le passage d'un premier fil d'alimentation électrique de la sonotrode, contactant la face du transducteur adjacente à la sonotrode 82. L'autre face est reliée électriquement à un deuxième fil d'alimentation.

Hormis l'évidement 114, la sonotrode 82 est dans l'exemple considéré symétrique de révolution autour de l'axe X.

Différents transducteurs peuvent être utilisés. Un transducteur 80 comportant une céramique piézoélectrique convenant à l'invention est par exemple celui commercialisé par la société Ferroperm sous la référence 26132. Il s'agit d'une céramique piezoélectrique PZ26 en forme d'anneau de diamètre extérieur 20mm, diamètre intérieur 3,8mm et d'épaisseur 2mm.

Le joint torique 110 repose sur un épaulement 116 du support 78, comme on peut le voir sur la figure 6 et le transducteur 80 appuie par sa face opposée à la sonotrode 82 sur le joint 110, à proximité de son bord radialement extérieur.

Le joint 110 permet un montage sans jeu de la sonotrode 82 et du transducteur 80 sur le support 78.

La sonotrode 82 comporte, à l'arrière, un premier tronçon cylindrique élargi 120, définissant un épaulement 125 sur lequel les dents 101 peuvent s'accrocher.

La sonotrode 82 se prolonge vers l'avant, au-delà de l'épaulement 125, par une portion tronconique 130 qui se raccorde, par un congé 131, à un deuxième tronçon cylindrique 132, d'axe X. Ce tronçon cylindrique 132 se raccorde par un congé 134 à une collerette d'extrémité 140 dont la face frontale, généralement perpendiculaire à l'axe X, définit la surface d'éjection 4 du produit.

Le diamètre D du premier tronçon cylindrique 120 est par exemple compris entre 18 et 22 mm, et vaut par exemple 20 mm. Ce diamètre D correspond par exemple sensiblement au plus grand diamètre du transducteur 80. Dans une variante, le transducteur 80 présente un diamètre de 15 mm.

La longueur *l₀* du tronçon cylindrique 120 est par exemple comprise entre 1,5 et 5,5 mm, et vaut par exemple 3,5 mm.

Le plus grand diamètre *D₂* de la portion tronconique 130 est par exemple compris entre 15,5 mm et 19,5 mm et vaut par exemple 17,5 mm et le plus petit diamètre *D₃* de la portion tronconique 130 est par exemple compris entre 8 et 12 mm, et vaut par exemple 10 mm. L'angle α au sommet de la portion tronconique 130 est de 30° dans l'exemple illustré.

Le rayon de courbure du congé 131 est par exemple compris entre 2 et 3 mm et vaut 2,5 mm dans l'exemple illustré et celui du congé 134 est par exemple compris entre 1 et 2 mm et vaut 1,5 mm dans l'exemple illustré.

La distance *l₁* entre l'épaulement 125 et la surface d'éjection 4, mesurée selon l'axe X, est par exemple comprise entre 13 et 17 mm et vaut par exemple 14.9 mm dans l'exemple illustré.

La distance 12 entre le sommet de la portion tronconique 130 et la surface d'éjection 4 est par exemple comprise entre 7 et 10 mm et vaut 8,4 mm dans l'exemple illustré.

La distance 13 entre l'extrémité arrière du deuxième tronçon cylindrique 132 et la surface d'éjection 4 est par exemple comprise entre 4 et 8 mm et vaut 5,9 mm dans l'exemple illustré.

La distance 14 entre l'extrémité avant du deuxième tronçon cylindrique 132 et la surface d'éjection 4 est par exemple comprise entre 1,5mm et 2,5 mm et vaut 2 mm dans l'exemple illustré.

Le diamètre *D₁* du deuxième tronçon cylindrique 132 est par exemple compris entre 4 et 6 mm et vaut 5,5 mm dans l'exemple illustré et l'épaisseur *e* de la collerette d'extrémité 140, mesurée selon l'axe X à proximité de son bord radialement extérieur, est par exemple compris entre 0,4 et 0,6 mm et vaut 0,5 mm dans l'exemple illustré.

Le diamètre *D₇* de la collerette d'extrémité est par exemple compris entre 7 et 13mm, étant de 10 mm dans l'exemple considéré.

La face arrière de la collerette d'extrémité 140 se termine, dans l'exemple considéré, perpendiculairement à l'axe X.

L'épaisseur de la collerette peut être constante à partir de sa périphérie sur une plage annulaire de largeur Δr, mesurée radialement, comprise entre 0.2 et 2 mm, étant de 0.5 mm dans l'exemple condidéré.

Le ratio *D₇*/*D₁* est par exemple compris entre 7/6 et 13/4 et le ratio *D₇*/*e* entre 70/6 et 130/4.

L'invention n'est pas limitée à la forme de collerette d'extrémité exemplifiée sur le dessin et d'autres formes sont possibles, par exemple une forme elliptique. Dans ce cas, le terme « diamètre » se rapporte à celui du cercle circonscrit à la collerette.

La sonotrode 82 est réalisée dans l'exemple considéré avec, à l'arrière, un embout 150 de raccordement au conduit d'alimentation 55, l'embout 150 étant par exemple monolithique, réalisé par usinage avec le reste de la sonotrode 82. Le tube 55 est par exemple inséré à force sur l'embout 150.

Un canal 160 d'alimentation en produit traverse la sonotrode 82 selon l'axe X. Une première portion 160a du canal 160 s'étend avec un diamètre intérieur constant, depuis l'extrémité inférieure 162 de l'embout 150 jusqu'à un point 165 situé au sein du deuxième tronçon cylindrique 132, où cette portion 160a il se raccorde à une portion rétrécie 160b par un alésage tronconique 160c.

Le diamètre intérieur *D₅* du canal 160, sur sa portion 160a de plus grand diamètre, est par exemple compris entre 1 et 3 mm et vaut 1,5 mm dans l'exemple illustré, et le diamètre *D₆* de la portion rétrécie 160b est par exemple compris entre 0,4 mm et 0,8 mm et vaut de préférence 0,6 mm.

La présence de la portion 160a de plus grand diamètre facilite l'usinage du canal 160 et permet de ne pas générer une perte de charge excessive. La présence de la portion rétrécie 160b conduit à des performances supérieures quant à la qualité du spray formé.

La longueur *l₇* de la portion rétrécie 160b, mesurée selon l'axe X, est par exemple comprise entre 2 et 3 mm et vaut par exemple 5 mm.

Le transducteur 80 est excité par exemple à une fréquence comprise entre 30 et 200 kHz, mieux 60 et 200 kHz, et la pompe 53 délivre à la surface d'éjection 4, par l'intermédiaire du canal 160 traversant la sonotrode 80, le produit à pulvériser.

La fréquence s'excitation du transducteur 80 peut être constante, ou mieux, asservie de façon à obtenir le maximum d'amplitude de vibration de la surface d'éjection et d'efficacité de pulvérisation.

Les composants électroniques du dispositif peuvent comporter un circuit électronique assurant cette fonction, de façon conventionnelle.

Le fonctionnement de la pompe 53 peut comporter, le cas échéant, à la fin de la pulvérisation, une inversion du sens de rotation du moteur pendant un court instant, afin de réaspirer le produit présent dans le canal et réduire le risque de séchage et de bouchage du canal.

Lors de l'application d'une tension électrique au transducteur 80 grâce à ces premier et deuxième fils d'alimentation, le transducteur 80 vibre dans l'exemple considéré radialement par rapport à l'axe X. Les vibrations ainsi générées se propagent avec une amplification de l'amplitude dans la sonotrode 82 jusqu'à la surface d'éjection 4, qui vibre axialement en fléchissant.

Sous l'effet des vibrations, la collerette d'extrémité 140 se déforme, et les oscillations de la collerette 140 provoquent l'éjection de gouttelettes de produit sur toute sa circonférence.

La taille moyenne des gouttelettes émises est par exemple comprise entre 20 et 30 µm.

Les gouttelettes de produit éjectées sont entraînées par le flux de l'air sortant de l'ouverture 90 vers la surface à traiter, et atteignant cette surface sous forme de gouttelettes.

Le débit de produit est par exemple compris entre 0.5 g/mn et 10 g/mn en fonction de la viscosité du produit à nébuliser.

Un dispositif selon l'invention peut permettre de former, dans un exemple, une tache de produit de 40 mm environ, pleine et homogène, sur la région à traiter.

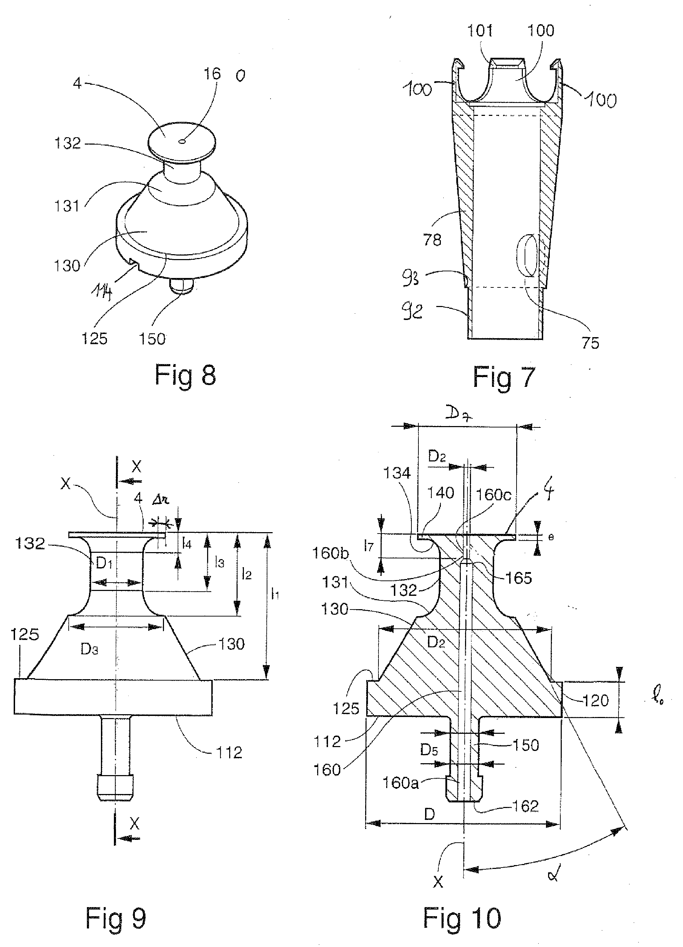

Dans l'exemple de la figure 10, les valeurs particulières des dimensions de la sonotrode ont été données pour une fréquence f de 100 kHz ;

Pour une fréquence différente f', les dimensions peuvent être modifiées d'un facteur f/f', en première approche.

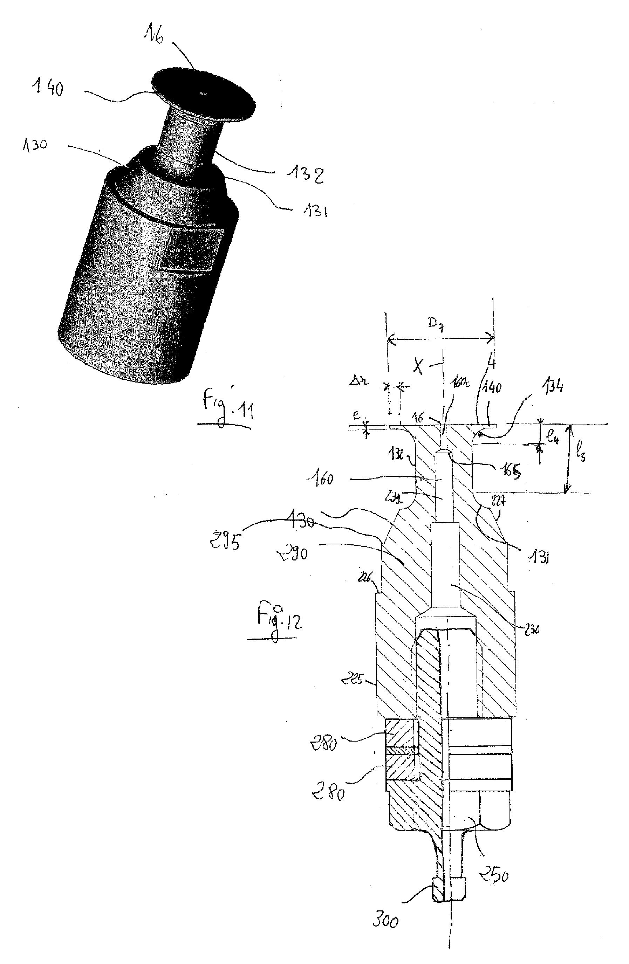

On a représenté à la figure 11 une variante de réalisation de la sonotrode, prévue pour fonctionner à une fréquence de 60 kHz. Cette sonotrode diffère de celle illustrée à la figure 10 par ses dimensions et par la forme du corps 290 situé en arrière de la portion cylindrique 132.

La sonotrode comporte un filetage intérieur 220 qui permet la fixation d'un boulon 250 de maintien d'un générateur de vibrations, composé par exemple de deux céramiques piézoélectriques 280 montées tête bêche.

La longueur *l₇* de la portion rétrécie 160c fait par exemple 3,5 mm. La longueur de la surface cylindrique 225 depuis la face d'extrémité opposée à la collerette 140 jusqu'à un épaulement 226 du corps 290 fait par exemple 18 mm, et la distance de l'épaulement 226 jusqu'à la base 295 d'une portion tronconique 227 adjacente à la portion cylindrique 132 fait par exemple 7 mm.

Le logement 229 recevant le boulon 250 communique avec deux alésages successifs 230 et 231 de diamètres respectifs décroissants, par exemple respectivement égaux à 4 et 2,5 mm.

Le boulon 250 est parcouru par une lumière centrale permettant d'amener le produit à pulvériser et peut comporter un embout 300 pour le raccordement du flexible 55.

Bien entendu, l'invention n'est pas limitée aux exemples de réalisation qui viennent d'être décrits.

Dans une variante non illustrée, l'alimentation en produit s'effectue par une aiguille qui délivre directement le produit à l'intérieur de la sonotrode, en retrait de l'orifice de sortie du produit.

La portion rétrécie du canal peut être formée non pas par un usinage de la sonotrode mais en rapportant sur celle-ci un réducteur d'écoulement, tel que par exemple un petit manchon inséré à force dans un canal de diamètre adapté de la sonotrode.

On peut donner au boîtier du dispositif 1 d'autres formes, notamment une forme de stylo.

Le boîtier manipulé par l'utilisateur peut être relié, le cas échéant, par un câble électrique, à un socle comprenant au moins l'alimentation électrique.

Dans une variante non illustrée, le canal d'alimentation débouche par plusieurs orifices sur la surface d'éjection. Ces orifices sont par exemple disposés en respectant une symétrie axiale. La portion rétrécie du canal peut se situer en amont des canaux communiquant avec les orifices ou en variante chaque embranchement du canal conduisant à un orifice comporte une portion rétrécie.

La surface d'éjection de la sonotrode peut avoir reçu un traitement de surface destiné par exemple à diminuer la tension de surface. Il peut s'agir par exemple d'un dépôt de PTFE ou d'un poli miroir.

Le cas échéant, le dispositif peut être agencé pour permettre un réglage du débord d de la surface d'éjection 4 par rapport à l'ouverture 90. Cela peut améliorer la focalisation du spray.

Dans une variante, le dispositif peut être utilisé pour pulvériser un produit dans l'atmosphère.

L'expression « comportant un » doit être comprise comme étant synonyme de « comportant au moins un », sauf si le contraire est spécifié.

Les plages de valeurs doivent s'entendre bornes incluses, sauf si le contraire est spécifié.

### Viscosité

La composition selon l'invention est liquide. Par liquide, on entend une composition qui s'écoule sous son propre poids.

La composition présente une viscosité allant de 5 à 500 mPa.s, de préférence de 10 à 400 mPa.s et plus préférentiellement allant de 20 à 150 mPa.s et de manière encore plus préférentielle allant de 25 à 100 mPa.s.

La viscosité est mesurée à 25 °C, avec un viscosimètre Rhéomat 180 équipé du mobile MK-R-1, 2 ou 3 suivant la gamme de viscosité et du godet de mesure correspondant MB-R-1, 2 ou 3, à une vitesse de rotation de 200 min-1, la mesure étant effectuée après 10 minutes de rotation (temps au bout duquel on observe une stabilisation de la viscosité et de la vitesse de rotation du mobile).

### Phase pulvérulente

La composition selon l'invention comprend une phase pulvérulente.

La phase pulvérulente peut notamment comprendre des pigments, des charges et/ou des nacres.

Selon un mode préféré de réalisation, la phase pulvérulente de la composition selon l'invention comprend des pigments.

Par « pigments », il faut comprendre des particules, minérales ou organiques, insolubles dans la phase organique liquide, destinées à colorer et/ou opacifier la composition.

Les pigments peuvent être des pigments minéraux ou organiques. Comme pigments, on peut utiliser les oxydes métalliques comme les oxydes de fer (notamment ceux de couleur jaune, rouge, brun, noir), les dioxydes de titane, l'oxyde de cérium, l'oxyde de zirconium, l'oxyde de chrome ; le violet de manganèse, l'ultramarine bleue, le bleu de prusse, le bleu outremer, le bleu ferrique et leurs mélanges.

On utilise de préférence des pigments d'oxydes de fer et/ou de dioxyde de titane.

Les pigments peuvent être traités avec un agent hydrophobe pour les rendre compatible avec la phase organique de la composition. L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.

Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium. L'acide aminé peut être par exemple la lysine, l'acide glutamique, l'alanine.

Le terme alkyl mentionné dans les composés cités précédemment désigne notamment un groupe alkyle ayant de 1 à 30 atomes de carbone, de préférence ayant de 5 à 16 atomes de carbone.

Des pigments traités hydrophobes sont notamment décrits dans la demande EP-A-1086683.

Les pigments peuvent être présents, dans la composition selon l'invention, en une teneur allant de 0,1 à 30% en poids, par rapport au poids total de la composition, en particulier allant de 1 % à 20% en poids, et plus préférentiellement allant de 3 % à 15% en poids.

Outre les pigments, la phase pulvérulente de la composition selon l'invention peut comprendre des charges et/ou des nacres.

Selon un mode préféré de réalisation, la composition selon l'invention peut comprendre des charges.

Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique (par exemple feuillet, cubique, hexagonale, orthorombique, etc) . On peut citer le talc, le mica, la silice, le kaolin, les poudres de polyamide (Nylon®) , les poudres de poly-β-alanine, les poudres de polyéthylène, les polyméthacrylates de méthyle, les poudres de polyuréthane telle que la poudre de copolymère de diisocyanate d'hexaméthylène et de triméthylol hexyl lactone vendue sous les dénominations PLASTIC POWDER D-400 par la société TOSHIKI, les poudres de polymères de tétrafluoroéthylène (Téflon®), les particules de cire micronisée notamment les microcires de Carnauba telles que celles commercialisées sous la dénomination de « MicroCare 350^{®} » par la société MICRO POWDERS, les microcires de cire synthétique telles que celles commercialisées sous la dénomination de « MicroEase 114S^{®} » par la société MICRO POWDERS, les microcires constituées d'un mélange de cire de Carnauba et de cire de polyéthylène telles que celles commercialisées sous les dénominations de « MicroCare 300^{®} » et « 310^{®} » par la société MICRO POWDERS, les microcires constituées d'un mélange de cire de Carnauba et de cire synthétique telles que celles commercialisées sous la dénomination de « MicroCare 325^{®} » par la société MICRO POWDERS, les microcires de polyéthylène telles que celles commercialisées sous les dénominations de « MicroPoly 200^{®} », « 220^{®} », « 220L^{®} », et « 250S^{®} », par la société MICRO POWDERS, et celles commercialisées sous la dénomination de « CERAPURE H5-C » par la société SHAMROCK, ou les microcires de polypropylène telle que celles commercialisées sous la dénomination « MATTEWAX » par la société MICRO POWDERS ; la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les poudres de résine de silicone, en particulier les poudres de silsesquioxane (poudres de résine de silicone notamment décrites dans le brevet EP 293795 ; Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; le sulfate de baryum et leurs mélanges.

Selon un mode préféré de réalisation, la composition selon l'invention peut comprendre une poudre de polytétrafluoroéthylène (PTFE), une poudre de polyamide (Nylon®) ou leur mélange.

Les charges peuvent être présentes dans la composition selon l'invention en une teneur totale allant de 0,1 % à 20 % en poids, par rapport au poids total de la composition, de préférence allant de 0,2 % à 15 % en poids, et préférentiellement allant de 0,5 % à 10 % en poids.

Outre les pigments et les charges, la phase particulaire de la composition selon l'invention peut comprendre des nacres.

Par « nacres », il faut comprendre des particules irisées, notamment produites par certains mollusques dans leur coquille ou bien synthétisées, insolubles dans le milieu de la composition.

Les nacres peuvent être choisies parmi les pigments nacrés blancs tels que l'oxychlorure de bismuth, le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Selon un mode préféré de réalisation, la phase pulvérulente de la composition selon l'invention peut représenter moins de 30 % en poids, par rapport au poids total de la composition, et en particulier de 0,1 à 30 % en poids, de préférence de 1 à 25 % en poids, plus préférentiellement de 3 à 20 % en poids, et plus préférentiellement encore de 10 à 20% en poids.

### Phase grasse

La composition selon l'invention peut comprendre une phase grasse.

La phase grasse peut notamment comprendre des huiles, des cires ou des corps gras pâteux.

### Huiles

La phase grasse peut comprendre au moins une huile volatile ou non volatile.

Par « huile volatile », on entend une huile susceptible de s'évaporer de la peau, en moins d'une heure à température ambiante et pression atmosphérique. Cette huile présente notamment une pression de vapeur, à température ambiante (25 °C) et pression atmosphérique (760 mm Hg) allant de 0,13 Pa à 40 000 Pa (10⁻³ à 300 mm de Hg), notamment allant de 1,3 Pa à 13 000 Pa (0,01 à 100 mm de Hg), et en particulier allant de 1,3 Pa à 1300 Pa (0,01 à 10 mm de Hg).

En outre, l'huile volatile a généralement un point d'ébullition, mesuré à pression atmosphérique, allant de 150 °C à 260 °C, et notamment allant de 170°C à 250°C.

Avantageusement, l'huile volatile contient une ou plusieurs huiles organiques volatiles dont le point éclair va de 30 °C à 102 °C, en particulier de 40 °C à 55 °C, et notamment de 40 °C à 50 °C et leurs mélanges.

Par « huile non volatile », on entend tout milieu susceptible de rester sur la peau pendant plusieurs heures. Une huile non volatile à en particulier une pression de vapeur à température ambiante et pression atmosphérique, non nulle inférieure à 0,001 mm de Hg (0,13 Pa).

Comme huile volatile utilisable dans l'invention, on peut citer les huiles volatiles hydrocarbonées ayant de 8 à 16 atomes de carbone, linéaires ou ramifiées, et leurs mélanges et notamment les alcanes ramifiés en C₈-C₁₆ comme les isoalcanes (appelées aussi isoparaffines) en C₈-C₁₆, l'isododécane, l'isohexadécane et par exemple les huiles vendues sous les noms commerciaux d'Isopars^{®} ou de Permethyls^{®}, les esters ramifiés en C₈-C₁₆ comme le néopentanoate d'iso-hexyle et leurs mélanges. On utilise en particulier l'isododécane.

Les huiles volatiles peuvent également être siliconées comportant éventuellement des groupes alkyles ou alkoxy pendants, ou en bout de chaîne siliconée, les huiles volatiles fluorées, et leurs mélanges.

Comme huile siliconée volatile utilisable dans l'invention, on peut citer les huiles de silicones linéaires, ramifiées ou cycliques ayant une viscosité à température ambiante inférieure à 8 mm²/s et ayant notamment de 2 à 7 atomes de silicium, ces silicones comportant éventuellement des groupes alkyle ou alkoxy ayant de 1 à 10 atomes de carbone pendants ou au bout de chaque silicone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthylhexyl trisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile fluorée n'a généralement pas de point éclair.

Comme huile volatile fluorée, on peut citer le nonafluoroéthoxybutane, le nonafluorométhoxybutane, le décafluoropentane, le tétradécafluorohexane, le dodécafluoropentane, et leurs mélanges.

L'huile volatile additionnelle peut être présente en une teneur allant de 1 % à 90 % en poids par rapport au poids total de la composition, de préférence de 2 % à 80 % en poids et de manière encore plus préférentielle de 3 à 65 %.

La composition peut comprendre au moins une autre huile non volatile.

Comme huiles non volatiles utilisables dans l'invention, on peut citer les huiles hydrocarbonées d'origine minérale ou synthétique telles que les hydrocarbures linéaires ou ramifiés comme l'huile de paraffine ou ses dérivés, l'huile de vaseline, les polydécènes, le polyisobutène hydrogéné tel que le Parléam commercialisé par la société NIPPON OIL FATS, le squalane d'origine synthétique ou végétale ; les huiles d'origine animale comme le perhydrosqualène ; les huiles d'origine végétale hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées, notamment les triglycérides d'acide gras notamment de 4 à 22 atomes de carbone, comme les triglycérides des acides heptanoïque, octanoïque, et des acides caprique/caprylique ou bien encore les triglycérides hydroxylés, tels que l'huile d'amande douce, de calophyllum, de palme, de pépins de raisin, de sésame, d'arara, de colza, de tournesol, de coton, d'abricot, de ricin, de luzerne, de courge, de cassis, de macadamia, de rosier muscat, de noisette, d'avocat, de jojoba, d'olive, de germes de céréales (maïs, blé, orge, seigle), de beurre de karité ; des esters d'acides gras, en particulier de 4 à 22 atomes de carbone, et notamment d'acide octanoïque, d'acide heptanoïque, d'acide lanolique, d'acide oléique, d'acide laurique, d'acide stéarique comme le dioctanoate de propylène glycol, le monoisostéarate de propylène glycol, le polyglycéryl 2 diisostéarate, le diheptanoate de néopentylglycol ; les esters de synthèse de formule R₁COOR₂ dans laquelle R₁ représente le reste d'un acide gras supérieur linéaire ou ramifié comportant de 7 à 40 atomes de carbone et R₂ représente une chaîne hydrocarbonée ramifiée contenant de 3 à 40 atomes de carbone, comme par exemple l'huile de Purcellin (octanoate de cétostéaryle), l'isononanoate d'isononyle, le néopentanoate d'isodécyle, le benzoate d'alcool en C₁₂ à C₁₅, le palmitate d'éthyl 2-hexyle, le stéarate d'octyl-2 dodécyle, l'érucate d'octyl-2 dodécyle, l'isostéarate d'isostéaryle, le benzoate d'octyl-2 dodécyle, des octanoates, décanoates ou ricinoléates d'alcools ou de polyalcools, le myristate d'isopropyle, le palmitate d'isopropyle, le stéarate de butyle, le laurate d'hexyle, l'adipate de diisopropyle, le palmitate de 2-éthyl-hexyle, le laurate de 2-hexyl-décyle, le palmitate de 2-octyl-décyle, le myristate de 2-octyldodécyle, le succinate de 2-diéthyl-hexyle, le malate de diisostéaryle, le triisostéarate de glycérine ou de diglycérine ; les esters hydroxylés comme le lactate d'isostéaryle, l'octylhydroxystéarate, l'hydroxystéarate d'octyldodécyle, le diisostéarylmalate, le citrate de triisocétyle ; le diisononanoate de diéthylèneglycol ; les esters du pentaérythritol ; les esters d'acides aromatiques et d'alcools comprenant 4 à 22 atomes de carbone, notamment le trimellitate de tridécyle ; les acides gras supérieurs en C₈-C₂₆ tels que l'acide oléique, l'acide linoléique, l'acide linolénique ou l'acide isostéarique ; les alcools gras supérieurs en C₈-C₂₆ tels que l'alcool oléique, l'alcool linoléique ou linolénique, l'alcool isostéarique ou l'octyl dodécanol ; les éthers de synthèse à au moins 7 atomes de carbone, les huiles siliconées telles que les polydiméthylsiloxanes (PDMS) liquides à température ambiante, linéaires, éventuellement phénylés tels que les phényltriméthicones, les phényl triméthylsiloxy diphénylsiloxanes, les diphényl diméthicones, les diphényl méthyldiphényl trisiloxanes, les 2-phényléthyl triméthylsiloxysilicates liquides, éventuellement substitués par des groupements aliphatiques et/ou aromatiques comme les groupes alkyle, alkoxy ou phényle, pendant et/ou en bout de chaîne siliconée, groupements ayant de 2 à 24 atomes de carbone et éventuellement fluorés, ou par des groupements fonctionnels tels que des groupements hydroxyle, thiol et/ou amine ; les polysiloxanes modifiés par des acides gras, des alcools gras ou des polyoxyalkylènes comme les diméthicones copolyols ou les alkylméthicones copolyols; les silicones fluorées liquides ; ou encore les triglycérides des acides caprylique/caprique comme ceux vendus par la société STEARINERIES Dubois ou ceux vendus sous les dénominations Miglyol 810, 812 et 818 par la société DYNAMIT NOBEL, et leurs mélanges.

Les huiles volatiles ou non volatiles additionnelles peuvent être présentes en une teneur allant de 1 % à 90 % en poids par rapport au poids total de la composition, de préférence de 2 % à 80 % en poids et de manière encore plus préférentielle de 3 à 65 %.

### Cires et pâteux

La phase grasse de la composition selon l'invention peut également comprendre au moins une cire, au moins une gomme et/ou au moins un corps gras pâteux, d'origine végétale, animale, minérale ou de synthèse, siliconé ou non.

Par « cire » au sens de la présente invention, on entend un composé gras lipophile, solide à température ambiante (25 °C), à changement d'état solide/liquide réversible, ayant une température de fusion supérieure à 30 °C pouvant aller jusqu'à 200 °C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires, au sens de la présente invention, peuvent être hydrocarbonées, siliconées et/ou fluorées, comportant éventuellement des fonctions ester ou hydroxyle. Elles sont notamment d'origine naturelle comme la cire d'abeilles éventuellement modifiée, la cire de Carnauba, de Candellila, d'Ouricoury, du Japon, de fibres de liège ou de canne à sucre, la cérésine, les cires de paraffine, de lignite, les cires microcristallines, la cire de lanoline, la cire de Montan, les ozokérites, les huiles hydrogénées comme l'huile de jojoba hydrogénée ou copolymérisation de l'éthylène, les cires obtenues par synthèse de Fischer-Tropsch, les esters d'acides gras et les glycérides concrets à 45 °C, les cires de silicone comme les alkyle, alkoxy et/ou esters de poly(di)méthylsiloxane solide à 45 °C, ayant de 10 à 45 atomes de carbone, certains acides gras comme l'acide stéarique, myristique, ou béhénique, et leurs mélanges.

La cire peut représenter de 0,01 à 20 % en poids, notamment de 0,1 à 10 % en poids, par rapport au poids total de la composition. Selon un mode de réalisation, la composition peut être exempte de cires.

Par « composé pâteux » au sens de l'invention, on entend un composé ayant un point de fusion allant de 25 à 60 °C, de préférence de 30 à 45 °C et une dureté allant de 0,001 à 0,5 MPa, de préférence de 0,005 à 0,4 MPa.

A titre d'exemple de corps gras pâteux, on peut citer les PDMS ayant des chaînes pendantes du type alkyle ou alkoxy ayant de 8 à 24 atomes de carbone comme le stéaryl diméthicone et notamment ceux vendus par DOW CORNING sous les références DC2503 ou DC05514 ; les esters d'alcool gras ou d'acide gras ayant de 20 à 25 atomes de carbone (un point de fusion notamment de 20 à 35 °C et/ou une viscosité à 40 °C allant de 0,1 à 40 Pa.s) comme les esters du cholestérol tels que les triglycérides d'origine végétale hydrogénés comme par exemple l'huile de ricin hydrogénée vendue sous le nom « Thixin R » par la société RHEOX, le polylaurate de vinyle, le propionate d'arachidyle, le triisostéaryl or cétyl citrate, le copolymère PVP/Eicosène ; les lanolines d'isopropyle, ayant une viscosité de 10 à 25 Pa.s, de préférence de 19 à 25 Pa.s et/ou un point de fusion de 25 à 45 °C et leurs mélanges.

La composition de l'invention peut également comprendre au moins une alkyl, alkoxy ou phényldiméthicone telle que, par exemple le produit vendu sous la dénomination de « Abil wax 2440^{®} » par la société GOLDSCHMIDT.

### Phase aqueuse

La composition selon l'invention peut comprendre une phase aqueuse comprenant de l'eau.

L'eau peut être une eau florale telle que l'eau de bleuet et/ou une eau minérale telle que l'eau de VITTEL, l'eau de LUCAS ou l'eau de LA ROCHE POSAY et/ou une eau thermale.

La phase aqueuse de la composition peut également comprendre des solvants organiques miscibles à l'eau à température ambiante (25 °C) comme par exemple les monoalcools ayant de 2 à 6 atomes de carbone tels que l'éthanol, l'isopropanol ; les polyols ayant notamment de 2 à 20 atomes de carbones, de préférence ayant de 2 à 10 atomes de carbone, et préférentiellement ayant de 2 à 6 atomes de carbone, tels que le glycérol, le propylène glycol, le butylène glycol, le pentylène glycol, l'hexylène glycol, le dipropylène glycol, le diéthylène glycol ; les éthers de glycol (ayant notamment de 3 à 16 atomes de carbone) tels que les alkyl(C₁-C₄)éther de mono, di- ou tripropylène glycol, les alkyl(C₁-C₄)éthers de mono, di- ou triéthylène glycol, et leurs mélanges.

La phase aqueuse peut comprendre en outre des agents de stabilisation, par exemple le chlorure de sodium, le dichlorure de magnésium et le sulfate de magnésium.

La phase aqueuse peut également comprendre tout composé hydrosoluble ou hydrodispersible compatible avec une phase aqueuse tels que des gélifiants, des polymères filmogènes, des épaississants, des tensioactifs, les actifs hydrosolubles et leurs mélanges.

Selon un mode préféré de réalisation, la composition selon l'invention comprend moins de x% en poids de gélifiants et/ou d'épaississants. Selon un mode plus préféré de réalisation, la composition selon l'invention est exempte de gélifiants et/ou d'épaississants.

La phase aqueuse (eau et éventuellement le(s) solvant(s) organique(s) miscible(s) à l'eau) peut être présente en une teneur allant de 5 % à 99,5 % en poids, notamment allant de 10 % à 90 % en poids, et en particulier allant de 15 % à 80 %, en poids par rapport au poids total de la composition.

### Colorants

La composition selon l'invention peut, en outre, comprendre au moins un colorant soluble.

Le colorant soluble peut être choisi parmi les colorants hydrosolubles, les colorants liposolubles et leurs mélanges.

Les colorants pouvant être utilisés dans les compositions selon l'invention sont des "systèmes de coloration directe" c'est-à-dire des agents de coloration ou mélange d'agents de coloration qui dès leur mise en contact avec le support de maquillage, généralement la peau, procurent instantanément un effet de coloration associé.

Sont ainsi exclus de cette définition, les composés qui exercent une action coloration différée sur la peau humaine à l'image des dérivés présentant un groupement cétone comme des hydroxyméthylcetones et en particulier la dihydroxyacétone (DHA) et également le méthylglyoxane. En effet, par opposition au système de coloration directe selon l'invention, ces composés provoquent un effet coloriel non immédiat.

Les colorants hydrosolubles selon l'invention peuvent être d'origine naturelle ou synthétique, d'origine végétale ou animale, en particulier naturels d'origine végétale.

A titre illustratif des agents de coloration naturels hydrosolubles susceptibles d'être mis en oeuvre selon l'invention, on peut particulièrement citer le caramel, le jus de betterave et le carmin, la bétanine (betterave), la chlorophylline cuivrée, le bleu de méthylène, les anthocyanines (enocianine, carotte noire, hibiscus, sureau) et la riboflavine. Les colorants hydrosolubles synthétiques sont par exemple le FDC Red 4, le DC Red 6, le DC Red 22, le DC Red 28, le DC Red 30, le DC Red 33, le DC Orange 4, le DC Yellow 5, le DC Yellow 6, le DC Yellow 8, le FDC Green 3, le DC Green 5, le FDC Blue 1.

Les colorants naturels liposolubles susceptibles d'être utilisés dans les compositions selon l'invention peuvent être choisis parmi le rouge Soudan, le β-carotène, les caroténoïdes, le lycopène, l'huile de palme, le rocou, le brun Soudan, le jaune quinoléique les xanthophylles (capsanthine, capsorubine, lutéine), et le curcumin. Comme exemple d'agent de coloration synthétique, on peut citer les colorants liposolubles, synthétiques tels que, par exemple, le DC Red 17, le DC Red 21, le DC Red 27, le DC Green 6, le DC Yellow 11, le DC Violet 2 et le DC Orange 5.

Comme autre agent de coloration naturel susceptibles de convenir à la présente invention, on peut plus particulièrement citer les anthocyanes de fleurs ou de fruits ou leurs dérivés, les flavonoïdes et tanins extraits de végétaux natifs ou fermentés, la juglone, la lawsone, les extraits de soja fermenté, d'algues, de champignons, de micro-organismes, les sels de Flavylium non substitués en position 3 tels que décrit dans le brevet EP 1 172 091, les extraits de Gesneria Fulgens, Blechum Procerum, Saxifraga et les pigments susceptibles d'être obtenus par extraction par un solvant organique ou hydro-organique d'un milieu de culture de micromycètes du type monascus Monascus.

Le ou les colorants solubilisés hydro ou liposolubles considérés dans le cadre de la présente invention peuvent être présents dans la composition en une teneur allant de 0,001 à 5 % en poids par rapport au poids total de la composition, notamment de 0,01 à 3 % en poids, et plus particulièrement de 0,025 à 1 % en poids.

### Additifs

La composition selon l'invention peut comprendre au moins un autre ingrédient cosmétique usuel pouvant être choisi notamment parmi les antioxydants, les parfums, les conservateurs, les neutralisants, les tensioactifs, les filtres solaires, les vitamines, les hydratants, les actifs antirides, les émollients, les actifs hydrophiles ou lipophiles, les agents anti-radicaux libres, les agents déodorants, les sequestrants, les agents filmogènes, et leurs mélanges.

### Galéniques

La composition selon l'invention peut se présenter sous diverses formes galéniques liquides.

Par exemple, elle peut se présenter sous forme d'une émulsion huile-dans-eau, eau-dans-huile ou multiple, une solution aqueuse ou hydro alcoolique et même sous forme biphasique.

Selon un mode préféré de réalisation, la composition selon l'invention se présente sous la forme d'une une solution aqueuse ou hydro alcoolique ou sous forme d'une émulsion huile-dans-eau, eau-dans-huile ou multiple, et plus préférentiellement eau-dans-huile.

Selon un mode préféré de réalisation, la composition selon l'invention se présente sous la forme d'une émulsion.

L'invention est présentée plus en détail dans les exemples ci-après.

### EXEMPLES :

### Exemple 1 : pulvérisation d'un fond de teint liquide

On a préparé un fond de teint liquide ayant la composition suivante :

| | | % massique |
|---|---|---|
| A1 | Cetyl PEG/PPG-10/1 dimethicone vendu sous la référence ABIL EM 90 par la société Goldschmidt | 1,44 |
| | Polyglyceryl 4 isostearate vendu sous la référence ISOLAN GI 34 par la société Goldschmidt | 0,48 |
| | Isododécane | 33,10 |
| A2 | Hectorite modifiée di stéaryl dimethyl ammonium vendu sous la référence Bentone 38 VCG par la société ELEMENTIS | 0,48 |
| A3 | Isododécane | 5,00 |
| | Oxyde de fer jaune enrobé de stéaroyl glutamate d'aluminium | 2,24 |
| | Oxyde de fer jaune enrobé de stéaroyl glutamate d'aluminium | 0,51 |
| | Oxyde de fer noir enrobé de stéaroyl glutamate d'aluminium | 0,45 |
| | Dioxyde de titane enrobé de stéaroyl glutamate d'aluminium | 6,80 |
| A4 | PTFE | 1,10 |
| | Poudre de nylon 12 | 0,90 |
| B | Eau déminéralisée | 34,3 |
| | Sulfate de magnésium | 0,56 |
| | Octane 1-2 diol | 0,24 |
| | Glycérol | 2,40 |
| C | Ethanol | 10,00 |
| | TOTAL | 100% |

### Mode opératoire

On pèse les constituants de la phase A1 dans le bécher principal puis on ajoute A2 sous agitation au Moritz à 1500 trs / min en maintenant à température ambiante.

La phase A3 est préparée séparément en broyant les pigments à la tri-cylindre puis elle est ajoutée sous agitation au mélange précédent.

On ajoute ensuite la phase A4 en maintenant l'agitation.

Pour préparer la phase aqueuse, on porte l'eau à l'ébullition puis on ajoute les autres constituants de la phase B.

L'émulsion se fait à température ambiante. Les deux phases doivent alors avoir une température voisine de l'ambiante.

On verse la phase aqueuse dans la phase grasse en augmentant au fur et à mesure la vitesse d'agitation du Moritz jusqu'à 2500 trs / min.

Après addition on laisse agiter pendant 10 min, puis on ajoute l'éthanol à température ambiante.

### Mesure de viscosité

La viscosité est mesurée à 25 °C, avec un viscosimètre Rhéomat 180 équipé du mobile MK-R2 et du godet de mesure MB-R2 d'un volume 60 ml, à une vitesse de rotation de 200 min-1, la mesure étant effectuée après 10 minutes de rotation (temps au bout duquel on observe une stabilisation de la viscosité et de la vitesse de rotation du mobile).

La viscosité de la composition de l'exemple 1, mesurée suivant ce protocole, est de 45 mPas.

### Essais de pulvérisation

La composition de l'exemple 1 est pulvérisée sur la moitié d'un visage à l'aide d'un appareil tel que décrit dans la demande de brevet WO 93/10910 et WO95/15822 de The Technology Partnership PLC, tel que décrit page 3 ligne 4 à page 4,1. 18.

Sur l'autre moitié du visage on applique la même composition à l'aide d'un coton.

On compare alors l'agrément à l'application et le résultat maquillage des deux modes d'application.

La pulvérisation du produit procure une sensation de légèreté et de fraîcheur qui est beaucoup moins importante avec l'application au coton.

La pulvérisation permet d'éviter de se tacher les doigts contrairement au coton.

Le résultat maquillage obtenu avec la pulvérisation est plus naturel, les imperfections en particulier les tâches et les pores sont moins marqués, le résultat est uniforme et ne nécessite pas de retouches aux doigts. La pulvérisation permet donc en outre d'éviter de se salir les mains.

La composition de l'exemple 1 peut aussi être pulvérisée au moyen du dispositif de pulvérisation de ladite composition, comprenant une sonotrode (82) d'axe longitudinal X, couplée à un transducteur piezoélectrique, cette sonotrode étant alimentée en composition via un canal débouchant selon l'axe X sur une collerette d'extrémité (140) définissant une surface d'éjection (4) de particules de produit, la collerette étant apte à fléchir sous l'effet des vibrations de la sonotrode de manière à pulvériser la composition sous forme de gouttelettes, tel que décrit de la page 5, ligne 35 à la page 20, ligne 44.

On obtient alors le même agrément à l'application et le même résultat maquillage que dans le test précédent.

### Exemple 2 : pulvérisation d'un fond de teint liquide

On a préparé un fond de teint liquide ayant la composition suivante :

| | | % massique |
|---|---|---|
| A1 | Bis-PEG/PPG-14/14 dimethicone dans Cyclopentasiloxane vendu sous la référence ABIL EM 97 par la société Goldschmidt | 1,80 |
| | Isostearyl diglyceryl succinate vendu sous la référence IMWITOR 780 K par la société SASOL | 0,60 |
| | Butyl paraben | 0,25 |
| A2 | Isododécane | 44,25 |
| | Hectorite modifiée di stéaryl dimethyl ammonium vendu sous la référence Bentone 38 VCG par la société ELEMENTIS | 0,60 |
| A3 | Cyclopentasiloxane | 5,00 |
| | Oxyde de fer jaune enrobé de stéaroyl glutamate d'aluminium | 2,24 |
| | Oxyde de fer jaune enrobé de stéaroyl glutamate d'aluminium | 0,51 |
| | Oxyde de fer noir enrobé de stéaroyl glutamate d'aluminium | 0,45 |
| | Dioxyde de titane enrobé de stéaroyl glutamate d'aluminium | 6,80 |
| A4 | PTFE | 1,40 |
| | Poudre de nylon 12 | 1,10 |
| B | Eau déminéralisée | 30,25 |
| | Methyl paraben | 0,25 |
| | Sulfate de Magnésium | 0,70 |
| | Phenoxy ethanol | 0,5 |
| | Octane 1-2 diol | 0,3 |
| | Glycérol | 3,00 |
| | TOTAL | 100% |

### Mode opératoire

On prépare la phase A1 en pesant les trois constituants et on chauffe le mélange jusqu'à 75 °C de manière à obtenir un milieu limpide puis on ajoute à température ambiante la phase A2 en agitant au Moritz à 1500 trs / min.

La phase A3 est préparée séparément en broyant les pigments à la tri-cylindre puis elle est ajoutée sous agitation au mélange précédent.

On ajoute ensuite la phase A4 en maintenant l'agitation.

Pour préparer la phase aqueuse on pèse les constituants de la phase B que l'on porte à l'ébullition.

L'émulsion se fait à température ambiante. Les deux phases doivent alors avoir une température voisine de l'ambiante.

On verse la phase aqueuse dans la phase grasse en augmentant au fur et à mesure la vitesse d'agitation du Moritz jusqu'à 2500 trs / min.

Après addition on laisse agiter pendant 10 min à température ambiante.

### Mesure de viscosité

La viscosité de la composition de l'exemple 2, mesurée suivant le protocole précédent, est de 45 mPas.

### Essais de pulvérisation

La composition de l'exemple 2 est pulvérisée sur la moitié d'un visage à l'aide d'un appareil tel que décrit dans la demande de brevet WO 93/10910 et WO95/15822 de The Technology Partnership PLC, tel que décrit page 3 ligne 4 à page 4,1. 18.

Sur l'autre moitié du visage on applique la même composition à l'aide d'un coton.

On compare alors l'agrément à l'application et le résultat maquillage des deux modes d'application.

La pulvérisation du produit procure une sensation de légèreté et de fraîcheur qui est beaucoup moins importante avec l'application au coton.

La pulvérisation permet d'éviter de se tacher les doigts contrairement au coton.

Le résultat maquillage obtenu avec la pulvérisation est plus naturel, les imperfections en particulier les tâches et les pores sont moins marqués, le résultat est uniforme et ne nécessite pas de retouches aux doigts. La pulvérisation permet donc en outre d'éviter de se salir les mains.

La composition de l'exemple 2 peut aussi être pulvérisée au moyen du dispositif de pulvérisation de ladite composition, comprenant une sonotrode (82) d'axe longitudinal X, couplée à un transducteur piezoélectrique, cette sonotrode étant alimentée en composition via un canal débouchant selon l'axe X sur une collerette d'extrémité (140) définissant une surface d'éjection (4) de particules de produit, la collerette étant apte à fléchir sous l'effet des vibrations de la sonotrode de manière à pulvériser la composition sous forme de gouttelettes, tel que décrit de la page 5, ligne 35 à la page 20, ligne 44.

On obtient alors le même agrément à l'application et le même résultat maquillage que dans le test précédent.

### Exemple 3: pulvérisation d'un fond de teint liquide

On a préparé un fond de teint liquide ayant la composition suivante :

| | | % massique |
|---|---|---|
| A1 | Cetyl PEG/PPG-10/1 dimethicone vendu sous la référence ABIL EM 90 par la société Goldschmidt | 1,44 |
| | Polyglyceryl 4 isostearate vendu sous la référence ISOLAN GI 34 par la société Goldschmidt | 0,48 |
| | Cyclopentasiloxane | 35,60 |
| A2 | Hectorite modifiée di stéaryl dimethyl ammonium vendu sous la référence Bentone 38 VCG par la société ELEMENTIS | 0,48 |
| A3 | Cyclopentasiloxane | 5,00 |
| | Oxyde de fer jaune enrobé de stéaroyl glutamate d'aluminium | 2,24 |
| | Oxyde de fer jaune enrobé de stéaroyl glutamate d'aluminium | 0,51 |
| | Oxyde de fer noir enrobé de stéaroyl glutamate d'aluminium | 0,45 |
| | Dioxyde de titane enrobé de stéaroyl glutamate d'aluminium | 6,80 |
| A4 | PTFE | 1,10 |
| | Poudre de nylon 12 | 0,90 |
| B | Eau déminéralisée | 20,00 |
| | Sulfate de magnésium | 0,56 |
| | Methyl paraben | 0,08 |
| | Phenoxy éthanol | 0,12 |
| | Octane 1-2 diol | 0,24 |
| | Glycérol | 2,40 |
| C | Cyclopentasiloxane | 21,60 |
| | TOTAL | 100% |

### Mode opératoire

On pèse les constituants de la phase A1 dans le bécher principal puis on ajoute A2 sous agitation au Moritz à 1500 trs / min en maintenant à température ambiante.

La phase A3 est préparée séparément en broyant les pigments à la tri-cylindre puis elle est ajoutée sous agitation au mélange précédent.

On ajoute ensuite la phase A4 en maintenant l'agitation.

Pour préparer la phase aqueuse on pèse les constituants de la phase B que l'on porte à l'ébullition.

L'émulsion se fait à température ambiante. Les deux phases doivent alors avoir une température voisine de l'ambiante.

On verse la phase aqueuse dans la phase grasse en augmentant au fur et à mesure la vitesse d'agitation du Moritz jusqu'à 2500 trs / min.

Après addition on laisse agiter pendant 10 min, puis on ajoute la phase C à température ambiante.

### Mesure de viscosité

La viscosité de la composition de l'exemple 3, mesurée suivant le protocole précédent, est de 60 mPas.

### Essais de pulvérisation

La composition de l'exemple 3 est pulvérisée sur la moitié d'un visage à l'aide d'un appareil tel que décrit dans la demande de brevet WO 93/10910 et WO95/15822 de The Technology Partnership PLC, tel que décrit page 3 ligne 4 à page 4,1. 18.

Sur l'autre moitié du visage on applique la même composition à l'aide d'un coton.

On compare alors l'agrément à l'application et le résultat maquillage des deux modes d'application.

La pulvérisation du produit procure une sensation de légèreté et de fraîcheur qui est beaucoup moins importante avec l'application au coton.

La pulvérisation permet d'éviter de se tacher les doigts contrairement au coton.

Le résultat maquillage obtenu avec la pulvérisation est plus naturel, les imperfections en particulier les tâches et les pores sont moins marqués, le résultat est uniforme et ne nécessite pas de retouches aux doigts. La pulvérisation permet donc en outre d'éviter de se salir les mains.

La composition de l'exemple 3 peut aussi être pulvérisée au moyen du dispositif de pulvérisation de ladite composition, comprenant une sonotrode (82) d'axe longitudinal X, couplée à un transducteur piezoélectrique, cette sonotrode étant alimentée en composition via un canal débouchant selon l'axe X sur une collerette d'extrémité (140) définissant une surface d'éjection (4) de particules de produit, la collerette étant apte à fléchir sous l'effet des vibrations de la sonotrode de manière à pulvériser la composition sous forme de gouttelettes, tel que décrit de la page 5, ligne 35 à la page 20, ligne 44.

On obtient alors le même agrément à l'application et le même résultat maquillage que dans le test précédent.

## Revendications

1. Ensemble cosmétique comprenant :
i) une composition cosmétique ou dermatologique contenue à l'intérieur d'un réservoir, ladite composition étant liquide et comprenant, dans un milieu physiologiquement acceptable, au moins une phase pulvérulente, et ladite composition présentant une viscosité allant de 5 à 500 mPa.s,
ii) un dispositif de pulvérisation de ladite composition, comprenant une sonotrode (82) couplée à un transducteur piezoélectrique et dont une surface d'éjection est alimentée en composition via un tube creux en couplage fluidique avec le réservoir et avec la sonotrode.

2. Ensemble cosmétique selon la revendication précédente, **caractérisée en ce qu'**une extrémité du tube creux est reliée à un canal traversant au moins en partie la sonotrode et débouchant sur une colerette d'extrémité définissant une surface d'éjection de la composition sous forme de gouttelettes, le canal débouchant sur ladite surface d'éjection à une distance non nulle d'un bord périphérique de cette dernière, le canal présentant une section transversale circulaire, sur toute sa longueur.

3. Ensemble cosmétique selon la revendication 2 **caractérisé en ce que** la sonotrode est d'axe longitudinal X, ledit canal débouchant sur la surface d'éjection selon ledit axe X.

4. Ensemble cosmétique selon la revendication 2, **caractérisé en ce que** le la collerette est apte à fléchir sous l'effet des vibrations de la sonotrode de manière à pulvériser la composition sous forme de goutelettes.

5. Ensemble cosmétique selon la revendication précédente, **caractérisé en ce que** l'épaisseur (e) de la collerette est comprise entre 0,4 mm et 0,6 mm.

6. Ensemble cosmétique selon l'une quelconque des revendications précédentes, le ratio *plus grand diamètre (D₇) de la collerette*/*épaisseur (e) de la collerette* étant compris entre 12 à 32.

7. Ensemble cosmétique selon l'une quelconque des revendications précédentes, la fréquence étant comprise entre 30 kHz et 200 kHz, de préférence entre 100 et 200 kHz, mieux 100 kHz +/-10%.

8. Ensemble cosmétique selon la revendication 2 ou l'une quelconque des revendications précédentes qui en dépendent **caractérisé en ce que** la sonotrode comporte également une portion de diamètre décroissant prolongée par une portion cylindrique (132) se raccordant à la collerette d'extrémité, le ratio *diamètre(D₇) de la collerette* / *diamètre (D₁) de la portion cylindrique* étant compris entre 12 et 32.

9. Ensemble cosmétique selon l'une quelconque des revendications précédentes, **caractérisé en ce que** la composition présente une viscosité allant de 10 à 400 mPa.s et plus préférentiellement allant de 20 à 150 mPa.s et de manière encore plus préférentielle allant de 25 à 100 mPa.s.

10. Ensemble cosmétique selon l'une quelconque des revendications précédentes, **caractérisé en ce que** la phase pulvérulente comprend des pigments.

11. Ensemble cosmétique selon la revendication précédente, **caractérisé en ce que** les pigments sont présents en une teneur allant de 0,1 à 30 % en poids, de préférence de 1 à 25 % en poids, plus préférentiellement de 3 à 20 % en poids.

12. Ensemble cosmétique selon l'une quelconque des revendications précédentes, **caractérisé en ce que** la phase pulvérulente comprend des charges.

13. Ensemble cosmétique selon la revendication précédente, **caractérisé en ce que** les charges sont présentes en une teneur allant de 0,1 % à 20 % en poids, par rapport au poids total de la composition, de préférence allant de 0,2 % à 15 % en poids, et préférentiellement allant de 0,5 % à 10 % en poids

14. Ensemble cosmétique selon l'une quelconque des revendications précédentes, **caractérisé en ce que** la composition se présente sous la forme d'une une solution aqueuse ou hydro alcoolique ou sous forme d'une émulsion huile-dans-eau, eau-dans-huile ou multiple, et plus préférentiellement eau-dans-huile.

15. Procédé de maquillage de la peau, en particulier pour obtenir un dépôt, notamment de maquillage, homogène, naturel et ne marquant pas les imperfections de relief de la peau comprenant au moins une étape de pulvérisation d'une composition cosmétique ou dermatologique au moyen d'un dispositif selon l'une quelconque des revendications 1 à 14.
